(19) Europäisches Patentamt
European Patent Office
Office européen des brevets

(11) EP 2 909 184 B1

(12) **FASCICULE DE BREVET EUROPEEN**

(45) Date de publication et mention de la délivrance du brevet:
**31.03.2021 Bulletin 2021/13**

(21) Numéro de dépôt: **13783284.6**

(22) Date de dépôt: **18.10.2013**

(51) Int Cl.:
*C07D 239/91* (2006.01) *C07D 409/04* (2006.01)
*C07D 409/14* (2006.01) *C07D 417/14* (2006.01)
*A61K 31/427* (2006.01) *A61K 31/517* (2006.01)
*A61K 31/381* (2006.01) *A61K 31/4439* (2006.01)
*A61K 31/506* (2006.01) *A61P 39/02* (2006.01)

(86) Numéro de dépôt international:
**PCT/EP2013/071863**

(87) Numéro de publication internationale:
**WO 2014/060586 (24.04.2014 Gazette 2014/17)**

(54) **NOUVEAUX COMPOSES AYANT UNE ACTIVITE PROTECTRICE VIS-A-VIS DE TOXINES AU MODE D'ACTION INTRACELLULAIRE**

NEUE VERBINDUNGEN, DIE EINE SCHUTZWIRKUNG GEGEN TOXINE ÜBER EINEN INTRAZELLULÄREN WIRKUNGSMECHANISMUS ENTFALTEN

NOVEL COMPOUNDS WITH PROTECTIVE ACTIVITY AGAINST TOXINS WITH INTRACELLULAR ACTION MODE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **19.10.2012 EP 12306297**

(43) Date de publication de la demande:
**26.08.2015 Bulletin 2015/35**

(73) Titulaires:
- **Commissariat à l'Énergie Atomique et aux Énergies Alternatives 75015 Paris (FR)**
- **Institut Curie 75248 Paris Cedex 05 (FR)**
- **CENTRE NATIONAL DE LA RECHERCHE SCIENTIFIQUE - CNRS 75016 Paris 16 (FR)**

(72) Inventeurs:
- **GILLET, Daniel F-92160 ANTONY (FR)**
- **BARBIER, Julien F-91190 Gif-sur-Yvette (FR)**
- **JOHANNES, Ludger F-92400 Courbevoie (FR)**
- **CINTRAT, Jean-Christophe F-91430 Igny (FR)**
- **NOEL, Romain F-22660 Trevou-Treguinec (FR)**

(74) Mandataire: **Gevers & Orès Immeuble le Palatin 2 3 Cours du Triangle CS 80165 92939 Paris La Défense Cedex (FR)**

(56) Documents cités:
- **JEWN GIEW PARK ET AL: "Chemical Structure of Retro-2, a Compound That Protects Cells against Ribosome-Inactivating Proteins", SCIENTIFIC REPORTS, vol. 2, 5 septembre 2012 (2012-09-05), XP055053611, DOI: 10.1038/srep00631 cité dans la demande**
- **NIKNAM K ET AL: "Silica-bonded N-propylsulfamic acid as a recyclable catalyst for the synthesis of 2,3-dihydroquinazolin-4(1H)-ones", CHINESE CHEMICAL LETTERS, ELSEVIER LTD, GB, vol. 22, no. 1, 1 janvier 2011 (2011-01-01), pages 69-72, XP027559177, ISSN: 1001-8417 [extrait le 2010-10-27]**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

- RENATO NOTO ET AL: "NMR analysis of restricted internal rotation in 2-substituted-2,3-dihydro-3- o -tolyl(chlorophenyl)-4(1 H )-quinazolinones", JOURNAL OF HETEROCYCLIC CHEMISTRY, vol. 33, no. 4, 1 juillet 1996 (1996-07-01), pages 1067-1071, XP055063338, ISSN: 0022-152X, DOI: 10.1002/jhet.5570330412
- LEI DI-WU ET AL: "Identification of CK2 inhibitors with new scaffolds by a hybrid virtual screening approach based on Bayesian model; pharmacophore hypothesis and molecular docking", JOURNAL OF MOLECULAR GRAPHICS AND MODELLING, ELSEVIER SCIENCE, NEW YORK, NY, US, vol. 36, 17 mars 2012 (2012-03-17), pages 42-47, XP028423237, ISSN: 1093-3263, DOI: 10.1016/J.JMGM.2012.03.004 [extrait le 2012-03-26]

## Description

La présente invention a pour objet une nouvelle famille de composés du type 2,3-dihydroquinazolin-4(1*H*)-one et leur utilisation en tant qu'inhibiteurs des effets toxiques des toxines à activité intracellulaire, comme par exemple la ricine, utilisant le transport rétrograde pour intoxiquer les cellules.

Les toxines à activité intracellulaire qui utilisent le transport rétrograde représentent un problème majeur de santé publique et sont associées à plus d'un million de décès chaque année dans le monde. Ces toxines sont notamment la ricine (produite dans les graines de la plante *Ricinus communis*), la toxine de Shiga et les toxines Shiga-like (Stxs) produite par *Shigella dysenteriae* (Stx) et *E. coli* (Stx1 et Stx2), la toxine cholérique (Ctx de *Vibrio cholerae* responsable du choléra), la toxine pertussique (*Bordetella pertussis* agent de la coqueluche), la cytotoxine subtilase et l'entérotoxine thermolabile (*E. coli*).

La ricine est une glycoprotéine de 66 kDa composée de deux chaînes polypeptidiques reliées par un pont disulfure **(Figure 1A**). La chaîne B (RTB, lectine de 262 résidus) permet à la toxine de se lier aux glycolipides et glycoprotéines des membranes cellulaires et d'assurer son entrée dans la cellule **(Figure 1A).** La chaîne A (RTA, 267 acides aminés) assure la fonction enzymatique N-glycosidase de la ricine, catalyse l'élimination de l'adénine 4324 de l'ARN 28S des cellules ciblées et provoque l'arrêt de la synthèse des protéines.

Les toxines de Shiga incluent la toxine de Shiga (Stx) produite par *Shigella dysenteriae* et les toxines Shiga-like 1 (Stx1) et 2 (Stx2) produites par les souches entérohémorragiques d'*E. coli.* Les toxines Stx et Stx1 sont à 99% identiques alors que les Stx1 et Stx2 partagent seulement 56% d'identité de leur séquence en acides aminés. La sous-unité A de la toxine (StxA) porte la même activité enzymatique que la ricine et cible de manière identique l'ARN 28S **(Figure 1B)**. La sous-unité B (StxB), qui se présente sous forme pentamérique, permet la liaison de la toxine avec la cellule *via* son interaction avec le globo-triaosylcéramide Gb3, ce qui assure son internalisation et son routage intracellulaire.

Après liaison à ses récepteurs membranaires, la ricine est internalisée dans ces cellules par de multiples voies d'endocytose pour atteindre le réseau *trans*-golgien où elle est acheminée vers le réticulum endoplasmique (RE) par transport rétrograde **(Figure 2A)**. Les toxines de Shiga sont internalisées quant à elles par une voie d'endocytose unique et atteignent également l'appareil de Golgi puis le réticulum endoplasmique (**Figure 2B**).

Les toxines sont alors partiellement dépliées et la chaîne/sous-unité A est transloquée dans le cytosol (Lord, J.M. et al., Biochemical Society Transactions 2003, 31, 1260). L'étape ultime de l'action de ces toxines a donc lieu dans le cytoplasme des cellules, les toxines se fixent sur les ribosomes avec une grande efficacité et clivent l'adénine 4324 de l'ARN 28S de la sous-unité 60S du ribosome. Cette dépurination de l'ARN 28S provoque l'arrêt de la synthèse des protéines et conduit à la mort cellulaire.

Pour contrer la menace posée par ces toxines, plusieurs types d'antitoxines ont été développés : anticorps neutralisants, inhibiteurs de l'activité enzymatique (petites molécules, analogues de substrat), mimes solubles de récepteurs et composés chimiques agissant sur les cellules ciblées par la toxine :

- Petites molécules inhibitrices de l'activité enzymatique de la ricine (Miller, D. et al., J. Med. Chem 2002, 45, 90 et Yan, X. et al., J. Mol. Biol. 1997, 266, 1043).
- Petites molécules inhibitrices de l'activité enzymatique de Stx (Brigotti M. et al., Nucleic Acids Res. 2000, 28 (12), 2383).
- Dérivés d'ARN : Comme la ricine reconnaît une séquence bien précise au niveau de l'ARN 28S, la boucle SRL (*Sarcin-Ricin Loop*)*,* des inhibiteurs ont été conçus en se basant sur cette séquence nucléotidique (Schramm et al. Biochemistry 2001, 40 (23), 6845; Roday, S. et al., Biochemistry 2004, 43, 4923; Hesselberth, J. R. et al., J. Biol. Chem. 2000, 275, 4937, Fan, S. et al., World J. Gastroenterol. 2008, 14, 6360).
- Anticorps dirigés contre la ricine (Lemley, P.V. et al., Hybridoma 1994, 13, 417 ; Guo, J.W. et al., Hybridoma 2005, 24, 263 ; Pelat, T. et al., BMC Biotechnol. 2009, 9, 60).
- Anticorps dirigés contre Stx (Sheoran A.S. et al., Infect Immun. 2005, 73, 4607-13 ; Tzipori S. et al., Clin. Microbiol. Rev. 2004 17, 926-41).
- Mimes solubles de récepteurs de la ricine (Perera L.P. et al., J Clin Microbiol. 1988, 26, 2127-31; Smith M.J. et al., Infect Immun. 2006, 74, 6992-8).
- Mimes solubles de récepteurs de Stx (Armstrong G.D. et al., J. Infect. Dis. 1995, 171, 1042-5 ; Trachtman H. et al., JAMA 2003, 290, 1337-44 ; Kitov P.I. et al., Nature 2000, 403, 669-72 ; Nishikawa K. et al., PNAS 2002, 99, 7669-74 ; Mulvey G.L. et al., J. Infect. Dis. 2003, 187, 640-9 ; Watanabe et al., J. Infect. Dis. 2004, 189, 360-8).
- Approche vaccinale pour contrer les effets de la ricine : un certain nombre de vaccins ont été décrits notamment dans des brevets et des publications scientifiques. Ils revendiquent une protection vis-à-vis de la ricine *via* l'administration d'une quantité immunogénique de dérivés de RTA ou RTB.
- Approche vaccinale pour contrer les effets de Stx : un vaccin efficace chez l'homme devrait conduire à la production d'anticorps prévenant la colonisation des bactéries dans le tractus intestinal ou neutralisant les toxines de Shiga

produites, afin d'empêcher le développement du syndrome hémolytique et urémique. Un tel vaccin n'est pas disponible à ce jour même si des travaux de recherche sont actuellement menés dans cette direction.

Ces dernières années, la recherche de nouvelles molécules visant à bloquer le routage intracellulaire des toxines à activité intracellulaire s'est accélérée. Le principal avantage de ce type de molécules est leur activité de type large spectre puisque ces molécules peuvent protéger efficacement les cellules contre les différentes toxines qui utilisent la voie rétrograde.

Cependant ces molécules, telles que la Bréfeldine A (Donta S.T. et al., J. Infect. Dis.1995 171, 721-4), Exo2 (Spooner R.A. et al., Biochem J. 2008, 414, 471-84 ;. Yarrow J.C. et al., C.C.H.T.S. 2003, 6, 279-86), Golgicide (Saenz J.B. et al., Nat. Chem. Biol. 2009, 5, 157-65) et autres (Saenz J.B. et al., Infect. Imm. 2007, 75, 4552-61), même si elles protègent les cellules contre l'action des toxines à activité intracellulaire, sont elles-mêmes cytotoxiques car elles ciblent des fonctions clé de l'homéostasie cellulaire et ne peuvent donc pas être utilisées dans un but thérapeutique.

D'autres molécules ont été identifiées notamment par criblage à haut débit en tant qu'inhibiteur de l'action de la ricine et/ou des Stx : les composés 134 et 75 (Saenz J.B. et al., Infect. Imm. 2007, 75, 4552-61) (*voir le schéma ci-après*). La cible cellulaire de ces molécules demeure inconnue ; elles semblent bloquer le transport de la ricine et des Stx à l'intérieur des cellules. Cependant, ces composés ne protègent pas les cellules épithéliales pulmonaires humaines A549 et aucune de ces molécules n'a démontré un effet protecteur chez l'animal. De plus, ces molécules modifient la morphologie de l'appareil de Golgi ce qui indique, d'une part, un mécanisme d'action différent et, d'autre part, une toxicité intrinsèque des composés.

Le criblage haut débit a également permis d'identifier les composés Retro-1 et Retro-2 (EP2145873, WO2009/153457, WO2009/153665, Stechmann B. et al., Cell 2010, 141, 231-42) en tant qu'inhibiteurs de la ricine et/ou de Stx ; ces composés sont capables de protéger les cellules épithéliales pulmonaires humaines A549 ; en outre, Retro-2 a montré une efficacité chez l'animal.

Une autre équipe de recherche a identifié de nouvelles molécules protectrices vis-à-vis de la ricine et des Stxs également lors d'un criblage à haut débit sur cellules Vero (Wahome P.G. et al., Toxicon 2010, 56, 313-23). Ces composés (CID 644401, CID 5737931 et CID 18576762) ne semblent pas aussi actifs (EC50 compris entre 25 et 60 μM) que les composés Retro-1 et Retro-2 et leur mode d'action reste inconnu.

**A. Inhibiteurs avec une cible cellulaire identifiée**

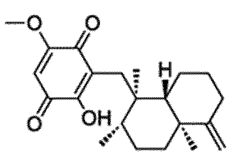

Ilimaquinone

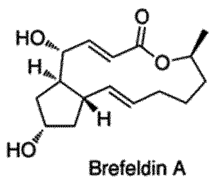

Brefeldin A

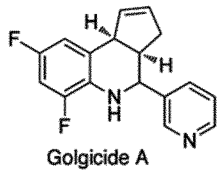

Golgicide A

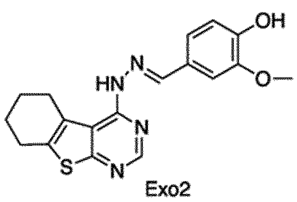

Exo2

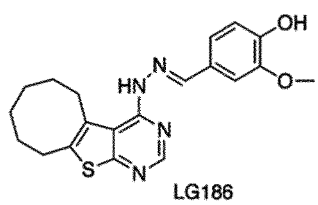

LG186

**B. Inhibiteurs dont la cible cellulaire est inconnue**

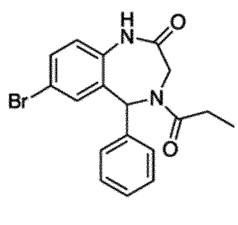
Retro-1

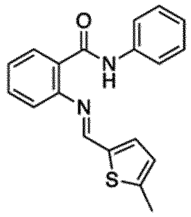
Retro-2

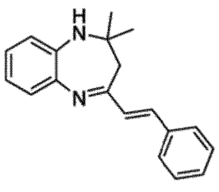
Compound 134

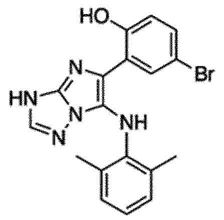
Compound 75

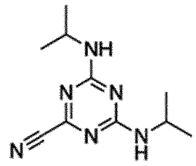
Compound 2
(CID 644401)

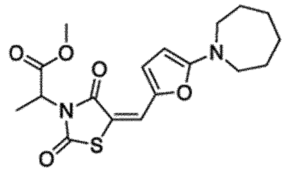
Compound 3
(CID 5737931)

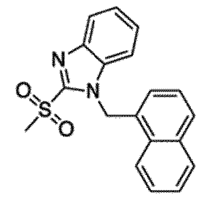
Compound 4
(CID 18576762)

*Petites molécules à activité intracellulaire inhibitrices de l'action de la ricine et/ou des Stx*

Très récemment, l'équipe de Park *et al.* [Park et al. Chemical Structure of Retro-2, a Compound That Protects Cells against Ribosome-Inactivating Proteins. Nature Scientific Reports. 2012. 2; 631] a étudié la structure chimique de Retro-2 et a mis en évidence la cyclisation spontanée de ce composé et montré que c'est sous cette forme cyclisée que Retro-2 exerçait une activité protectrice des cellules contre les toxines.

Dans le cadre de ses recherches sur des composés bloquant le transport rétrograde et plus particulièrement d'études sur des composés dérivés de Retro-2 cyclisé menées parallèlement aux travaux précités, la Demanderesse a identifié une nouvelle famille de composés dérivés de 2,3-dihydroquinazolin-4(1*H*)-one qui présentent une activité biologique supérieure à celle de Retro-2 cyclisée et donc un intérêt marqué pour la prévention et/ou le traitement des intoxications à au moins une toxine à mode d'action intracellulaire utilisant le transport rétrograde pour infecter les cellules eucaryotes de mammifères.

L'objet de la présente invention est défini par les revendications 1 à 9.

Ainsi, la présente description se rapporte à des composés de formule générale (I) :

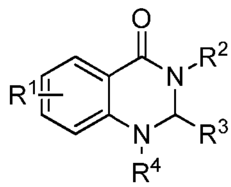
(I)

où

**$R^1$** représente un atome d'hydrogène, un atome d'halogène ou un radical alcoxy de 1 à 3 atomes de carbone ;
**$R^2$** représente :

- un radical phényle, éventuellement substitué par : un radical phényle, un groupe - $CF_3$, un atome d'halogène, un groupe -$SO_2$-phényle ou un groupe -S-X ou -O-X, X étant un radical alkyle de 1 à 4 atomes de carbone, de préférence un radical méthyle, un alcoxy de 1 à 4 atomes de carbone ou un PEG de formule -$(CH_2$-$CH_2$-$O)_n$-R avec n valant 1 à 10, de préférence n vaut 1, et R représente un atome d'hydrogène ou un radical méthyle, de préférence R est un radical méthyle, ; un groupe -CO-Y ou -CO-O-Y, Y étant choisi parmi un atome d'hydrogène, un radical alkyle de 1 à 4 atomes de carbone, un cycle phényle ou un groupe allyle (-$CH_2$-CH=$CH_2$) ;

  - un hétérocycle ou un hétérobicycle de 5 à 10 atomes comprenant un ou deux atomes d'azote, de préférence, un radical pyridine ;
  - un cycle adamantyl, éventuellement substitué par une fonction hydroxyle ;

**$R^3$** représente :

- un hétérocycle aromatique de 5 à 6 atomes qui peut être choisi parmi un cycle thiophène, furane, pyrrole, pyrroline, pyrrolidine, dioxolane, oxazole, thiazole, imidazole, imidazoline, imidazolidine, pyrazole, isoxazole, isothiazole, pyrane, pyridine, pipéridine, dioxane, morpholine, pyridazine, pyrimidine, pyrazine, de préférence, un cycle thiophène ou pyridine ;
  ledit hétérocycle est substitué par :

    * un radical alkyle de 1 à 3 atomes de carbone, préférentiellement méthyle ;
    * un atome d'halogène ;
    * un radical phényle éventuellement substitué par un radical alcoxy de 1 à 3 atomes de carbone, un groupe -CN, un groupe $-NO_2$ ou un groupe -COX ou -COOX avec X un radical alkyle de 1 à 4 atomes de carbone ou une combinaison de ces substituants, de préférence, un radical méthyle ;
    * un groupe -SY, Y étant un radical alkyle de 1 à 4 atomes de carbone ou un radical phényle ;
    * un groupe -CN ;
    * un groupe $-CH_2-N_3$ ;
    * un hétérocycle aromatique de 5 ou 6 atomes qui peut être choisi parmi un cycle furane, thiophène, pyrrole, pyrroline, pyrrolidine, dioxolane, oxazole, thiazole, imidazole, imidazoline, imidazolidine, pyrazole, isoxazole, isothiazole, pyrane, pyridine, pipéridine, dioxane, morpholine, pyridazine, pyrimidine, pyrazine, de préférence, ledit hétérocycle de 5 ou 6 atomes est un thiophène, une pyridine, un furane, un thiazole ; éventuellement substitué par au moins un radical alkyle de 1 à 3 atomes de carbone et/ou un groupe $-CH_2-N_3$ ;

- un radical phényle ; éventuellement substitué par un radical alcoxy de 1 à 3 atomes de carbone ou un groupe $NMe_2$ ;

**$R^4$** représente un atome d'hydrogène ou un radical méthyle ;
à l'exception du composé tel que $R^1$ est un atome d'hydrogène, $R^2$ un radical phényle, $R^3$ un radical 5-méthylthiophèn-2-yl et $R^4$ un atome d'hydrogène,
et leurs sels pharmaceutiquement acceptables.

La présente description se rapporte aux composés de formule générale (I) en tant que tels et également à leur utilisation pour la prévention et/ou le traitement des désordres induits par les toxines à activité intracellulaire utilisant le transport rétrograde.

La présente description se rapporte également à une méthode de prévention et/ou de traitement des désordres induits par les toxines à activité intracellulaire utilisant le transport rétrograde et comprenant l'administration d'une quantité efficace d'au moins un composé de formule générale (I).

De préférence, les composés de formule générale (I) suivants :

3-(4-Chlorophenyl)-2-(5-methylthiophen-2-yl)-2,3-dihydroquinazolin-4(1*H*)-one;
$R^1$ = -H ; $R^2$ = pyridinyl ; $R^3$ = 3,4-diméthoxyphényl et $R^4$ =-H ;
$R^1$ = -H ; $R^2$ = méthoxyphényl ; $R^3$ = pyridinyle et $R^4$ = -H ;
$R^1$ = -H ; $R^2$ = phényle ; $R^3$ = 6-methyl-2-pyridinyle et $R^4$ = -H ;
$R^1$ = -H ; $R^2$ = méthoxyphényle ; $R^3$ = diméthylaminophényle et $R^4$ = -H ;
R 1 = -H ; $R^2$ = phényle ; $R^3$ = furanyle et $R^4$ = -H ;
$R^1$ = -H ; $R^2$ = phényle-phényle ; $R^3$ = méthoxyphényle et $R^4$ = -H ;
$R^1$ = -H ; $R^2$ = phényle ; $R^3$ = diméthylaminophényle et $R^4$ = -H ;
$R^1$ = -H ; $R^2$ = phényle ; $R^3$ = phényle et $R^4$ = -H ;
$R^1$ = -H ; $R^2$ = 4-chlorophényle ; $R^3$ = 5-methyl-2-thienyl et $R^4$ = -H ;
$R^1$ = -H ; $R^2$ = phenyl ; $R^3$ = méthoxyphényle et $R^4$ = -H ;
et ceux tels que $R^2$ est un phényle substitué et $R^3$ un cycle furane, un phényle substitué ou une pyridine ;
en tant que tels sont exclus de l'objet de la présente description ; en revanche, leur utilisation à des fins thérapeutiques, en particulier, pour la prévention et/ou le traitement des désordres induits par les toxines à activité intracellulaire utilisant le transport rétrograde est bien un objet de la présente description.

Par sel pharmaceutiquement acceptable des composés de formule générale (I), on entend les chlorhydrates, bromhydrates, sulfates ou bisulfates, phosphates ou hydrogénophosphates, acétates, oxalates, benzoates, succinates, fumarates, maléates, lactates, citrates, tartrates, gluconates, méthanesulphonates, benzène-sulphonates et paratoluène- sulphonates.

Par atome d'halogène, on entend les éléments chimiques du groupe VII du tableau périodique des éléments, notamment, le fluor, le chlore, le brome et l'iode.

Le terme radical alkyle de 1 à 3 ou 4 atomes de carbone désigne un radical hydrogénocarboné, linéaire ou ramifié ; on peut citer par exemple le méthyle, l'éthyle, le propyle, l'isopropyle ou le tertiobutyle.

Par radical alcoxy de 1 à 3 ou 4 atomes de carbone, on entend un radical - $OC_nH_{2n+1}$, n étant un nombre entier compris entre 1 et 3 ou 4 ; on peut citer par exemple le radical méthoxy, éthoxy, propyloxy, isopropyloxy. De préférence n vaut 1.

Plus particulièrement, les composés de formule générale (I) sont choisis parmi le Tableau I ci-après (les composés 1 à 34 ne font pas partie de l'invention):

| molécule | Composé | structure | Nom |
|---|---|---|---|
| RN-1-013 | 1 | 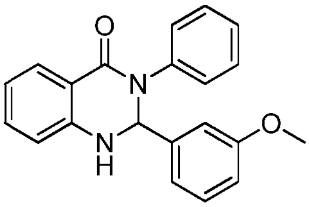 | 2-(3-Methoxyphenyl)-3-phenyl-2,3-dihydroquinazolin-4(1*H*)-one |
| RN-1-019 | 2 | 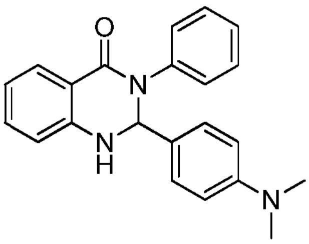 | 2-(4-(Dimethylamino)phenyl)-3-phenyl-2,3-dihydroquinazolin-4(1*H*)-one |
| RN-1-021 | 3 | 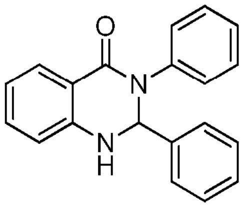 | 2,3-Diphenyl-2,3-dihydroquinazolin-4(1*H*)-one |
| RN-1-027 | 4 | 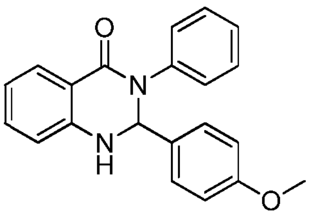 | 2-(4-Methoxyphenyl)-3-phenyl-2,3-dihydroquinazolin-4(1*H*)-one |
| RN-1-066 | 5 | 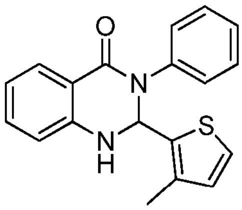 | 2-(3-Methylthiophen-2-yl)-3-phenyl-2,3-dihydroquinazolin-4(1*H*)-one |

(suite)

| molécule | Composé | structure | Nom |
|---|---|---|---|
| RN-1-067 | 6 | 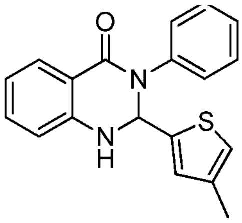 | 2-(4-Methylthiophen-2-yl)-3-phenyl-2,3-dihydroquinazolin-4(1*H*)-one |
| RN-1-068 | 7 | 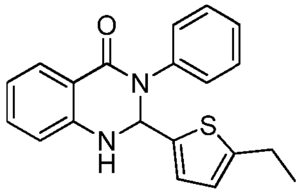 | 2-(5-Ethylthiophen-2-yl)-3-phenyl-2,3-dihydroquinazolin-4(1*H*)-one |
| RN-1-086 | 8 | 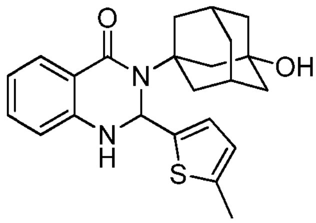 | 3-((5R,7S)-3-Hydroxyadamantan-1-yl)-2-(5-methylthiophen-2-yl)-2,3-dihydroquinazolin-4(1*H*)-one |
| RN-1-100 | 9 | 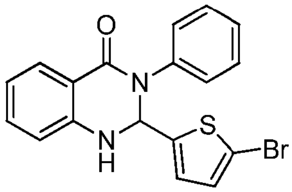 | 2-(5-Bromothiophen-2-yl)-3-phenyl-2,3-dihydroquinazolin-4(1*H*)-one |
| RN-1-118 | 10 | 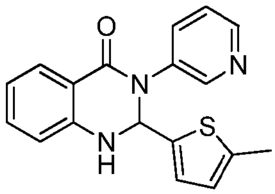 | 2-(5-Methylthiophen-2-yl)-3-(pyridin-3-yl)-2,3-dihydroquinazolin-4(1*H*)-one |
| RN-1-120 | 11 | 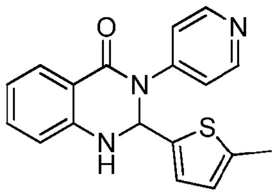 | 2-(5-Methylthiophen-2-yl)-3-(pyridin-4-yl)-2,3-dihydroquinazolin-4(1*H*)-one |
| RN-1-132 | 12 | 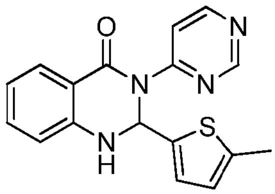 | 2-(5-Methylthiophen-2-yl)-3-(pyrimidin-4-yl)-2,3-dihydroquinazolin-4(1*H*)-one |

(suite)

| molécule | Composé | structure | Nom |
|---|---|---|---|
| RN-1-148 | 13 | 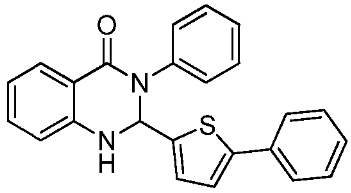 | 3-Phenyl-2-(5-phenylthiophen-2-yl)-2,3-dihydroquinazolin-4(1*H*)-one |
| RN-1-149 | 14 | 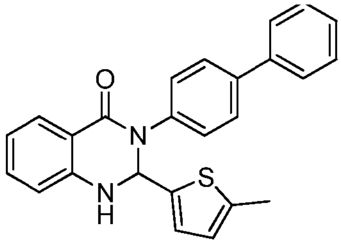 | 3-([1,1'-Biphenyl]-4-yl)-2-(5-methylthiophen-2-yl)-2,3-dihydroquinazolin-4(1*H*)-one |
| RN-1-151 | 15 | 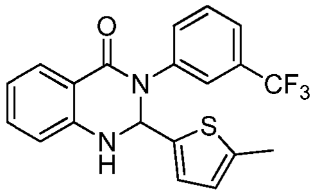 | 2-(5-Methylthiophen-2-yl)-3-(3-(trifluoromethyl)phenyl)-2,3-dihydroquinazolin-4(1*H*)-one |
| RN-1-162 | 16 | 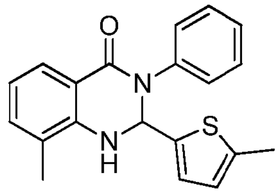 | 8-Methyl-2-(5-methylthiophen-2-yl)-3-phenyl-2,3-dihydroquinazolin-4(1*H*)-one |
| RN-1-173 | 17 | 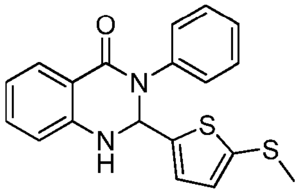 | 2-(5-(Methylthio)thiophen-2-yl)-3-phenyl-2,3-dihydroquinazolin-4(1*H*)-one |
| RN-1-174 | 18 | 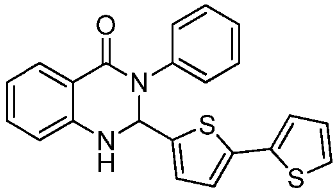 | 2-([2,2'-Bithiophen]-5-yl)-3-phenyl-2,3-dihydroquinazolin-4(1*H*)-one |
| RN-1-175 | 19 | 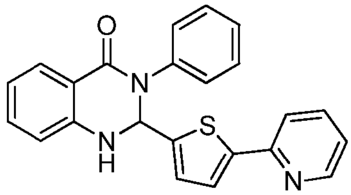 | 3-Phenyl-2-(5-(pyridin-2-yl)thiophen-2-yl)-2,3-dihydroquinazolin-4(1*H*)-one |

(suite)

| molécule | Composé | structure | Nom |
|---|---|---|---|
| RN-1-176 | 20 | 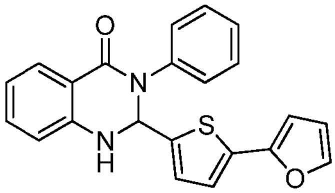 | 2-(5-(Furan-2-yl)thiophen-2-yl)-3-phenyl-2,3-dihydroquinazolin-4(1*H*)-one |
| RN-1-177 | 21 | 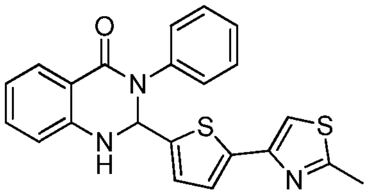 | 2-(5-(2-Methylthiazol-4-yl)thiophen- 2-yl)-3-phenyl-2,3-dihydroquinazolin-4(1*H*)-one |
| RN-2-120 | 22 | 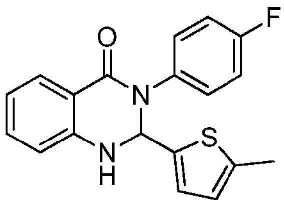 | 3-(4-Fluorophenyl)-2-(5-methylthiophen-2-yl)-2,3-dihydroquinazolin-4(1*H*)-one |
| RN-2-137 | 23 | 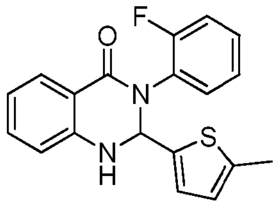 | 3-(2-Fluorophenyl)-2-(5-methylthiophen-2-yl)-2,3-dihydroquinazolin-4(1*H*)-one |
| RN-2-161 | 24 | 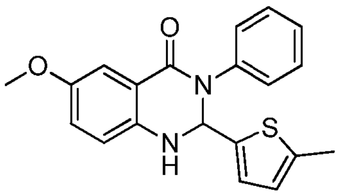 | 6-Methoxy-2-(5-methylthiophen-2-yl)-3-phenyl-2,3-dihydroquinazolin-4(1*H*)-one |
| RN-2-162 | 25 | 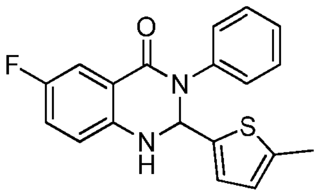 | 6-Fluoro-2-(5-methylthiophen-2-yl)-3-phenyl-2,3-dihydroquinazolin-4(1*H*)-one |
| RN-2-163 | 26 | 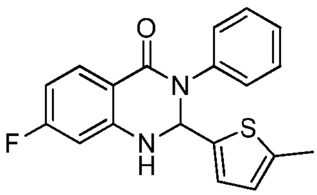 | 7-Fluoro-2-(5-methylthiophen-2-yl)-3-phenyl-2,3-dihydroquinazolin-4(1*H*)-one |

(suite)

| molécule | Composé | structure | Nom |
|---|---|---|---|
| RN-2-177 | 27 | 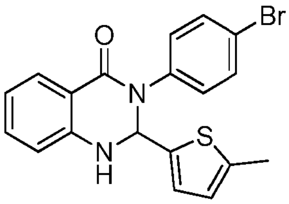 | 3-(4-Bromophenyl)-2-(5-methylthiophen-2-yl)-2,3-dihydroquinazolin-4(1*H*)-one |
| RN-2-178 | 28 | 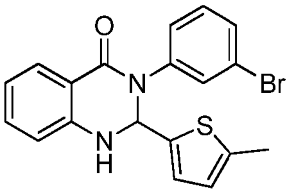 | 3-(3-Bromophenyl)-2-(5-methylthiophen-2-yl)-2,3-dihydroquinazolin-4(1*H*)-one |
| RN-2-180 | 29 | 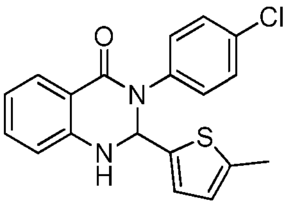 | 3-(4-Chlorophenyl)-2-(5-methylthiophen-2-yl)-2,3-dihydroquinazolin-4(1*H*)-one |
| RN-2-181 | 30 | 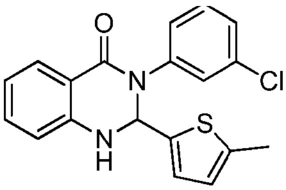 | 3-(3-Chlorophenyl)-2-(5-methylthiophen-2-yl)-2,3-dihydroquinazolin-4(1*H*)-one |
| RN-2-183 | 31 | 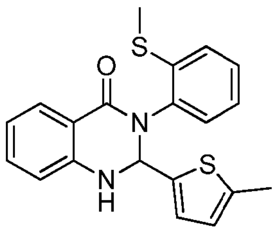 | 3-(2-(Methylthio)phenyl)-2-(5-methylthiophen-2-yl)-2,3-dihydroquinazolin-4(1*H*)-one |
| RN-2-182 | 32 | 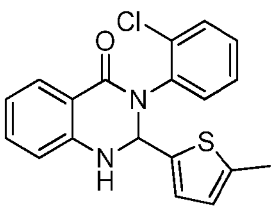 | 3-(2-Chlorophenyl)-2-(5-methylthiophen-2-yl)-2,3-dihydroquinazolin-4(1*H*)-one |
| RN-2-199 | 33 | 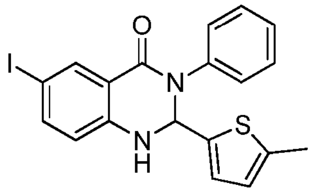 | 6-Iodo-2-(5-methylthiophen-2-yl)-3-phenyl-2,3-dihydroquinazolin-4(1*H*)-one |

(suite)

| molécule | Composé | structure | Nom |
|---|---|---|---|
| RN-3-121 | 34 | 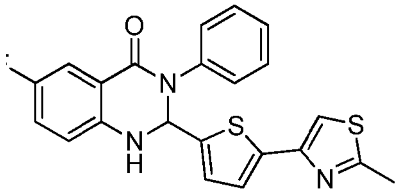 | 6-fluoro-2-(5-(2-methylthiazol-4-yl) thiophen-2-yl)-3-phenyl-2,3-dihydroquinazolin-4(1H)-one |
| RN-1-181 | 35 | 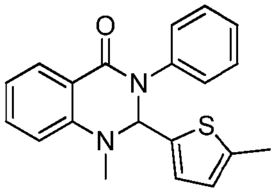 | 1-Methyl-2-(5-methylthiophen-2-yl)-3-phenyl-2,3-dihydroquinazolin-4 (1*H*)-one |
| RN-1-186 | 36 | 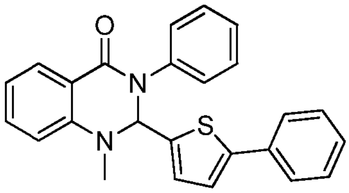 | 1-Methyl-3-phenyl-2-(5-phenylthiophen-2-yl)-2,3-dihydroquinazolin-4(1*H*)-one |
| RN-1-192 | 37 | 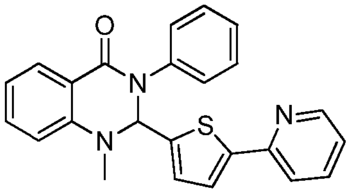 | 1-Methyl-3-phenyl-2-(5-(pyridin-2-yl) thiophen-2-yl)-2,3-dihydroquinazolin-4 (1*H*)-one |
| RN-1-196 | 38 | 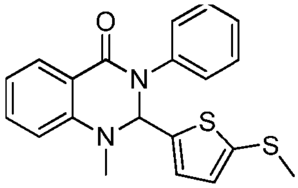 | 1-Methyl-2-(5-(methylthio)thiophen-2-yl)-3-phenyl-2,3-dihydroquinazolin-4 (1*H*)-one |
| RN-1-197 | 39 | 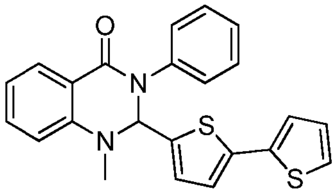 | 2-([2,2'-Bithiophen]-5-yl)-1-methyl-3-phenyl-2,3-dihydroquinazolin-4 (1*H*)-one |
| RN-2-001 | 40 | 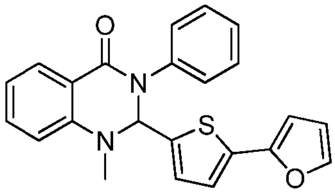 | 2-(5-(Furan-2-yl)thiophen-2-yl)-1-methyl-3 -phenyl-2,3 -dihydroquinazolin-4(1*H*)-one |

(suite)

| molécule | Composé | structure | Nom |
|---|---|---|---|
| RN-2-005 | 41 | 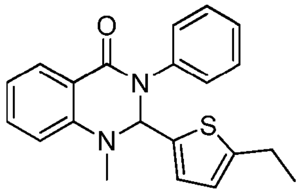 | 2-(5-Ethylthiophen-2-yl)-1-methyl-3-phenyl-2,3-dihydroquinazolin-4(1*H*)-one |
| RN-2-015 | 42 | 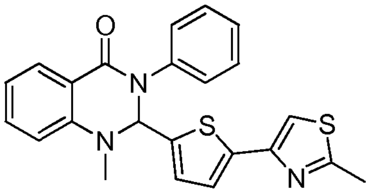 | 1-Methyl-2-(5-(2-methylthiazol-4-yl)thiophen-2-yl)-3-phenyl-2,3-dihydroquinazolin-4(1*H*)-one |
| RN-2-016 | 43 | 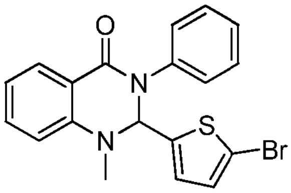 | 2-(5-Bromothiophen-2-yl)-1-methyl-3-phenyl-2,3-dihydroquinazolin-4(1*H*)-one |
| RN-2-019 | 44 | 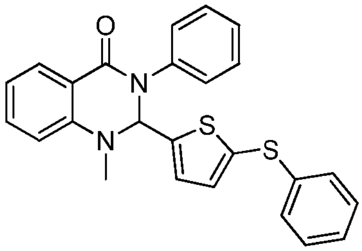 | 1-Methyl-3-phenyl-2-(5-(phenylthio)thiophen-2-yl)-2,3-dihydroquinazolin-4(1*H*)-one |
| RN-2-029 | 45 | 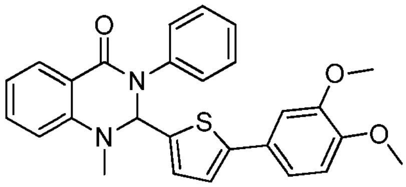 | 2-(5-(3,4-Dimethoxyphenyl)thiophen-2-yl)-1-methyl-3-phenyl-2,3-dihydroquinazolin-4(1*H*)-one |
| RN-2-032 | 46 | 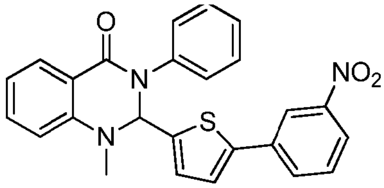 | 1-Methyl-2-(5-(3 -nitrophenyl)thiophen-2-yl)-3-phenyl-2,3-dihydroquinazolin-4(1*H*)-one |
| RN-2-034 | 47 | 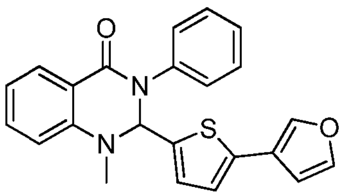 | 2-(5-(Furan-3-yl)thiophen-2-yl)-1-methyl-3-phenyl-2,3-dihydroquinazolin-4(1*H*)-one |

(suite)

| molécule | Composé | structure | Nom |
|---|---|---|---|
| RN-2-049 | 48 | 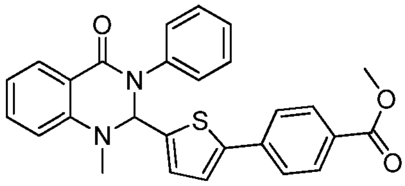 | Methyl 4-(5-(1-methyl-4-oxo-3 -phenyl-1,2,3,4-tetrahydroquinazolin-2-yl) thiophen-2-yl)benzoate |
| RN-2-050 | 49 | 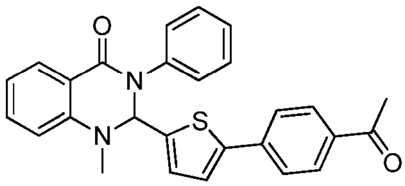 | 2-(5-(4-Acetylphenyl)thiophen-2-yl)-1-methyl-3-phenyl-2,3-dihydroquinazolin-4(1*H*)-one |
| RN-2-053 | 50 | 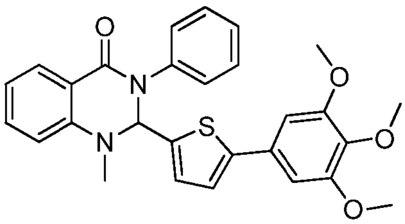 | 1-Methyl-3-phenyl-2-(5-(3,4,5-trimethoxyphenyl)thiophen-2-yl) -2,3-dihydroquinazolin-4(1*H*)-one |
| RN-2-057 | 51 | 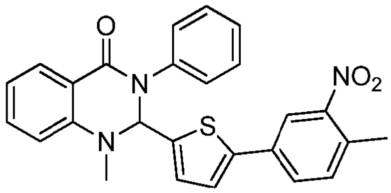 | 1-methyl-2-(5-(4-methyl-3-nitrophenyl) thiophen-2-yl)-3-phenyl-2,3-dihydroquinazolin-4(1 H)-one |
| RN-2-059 | 52 | 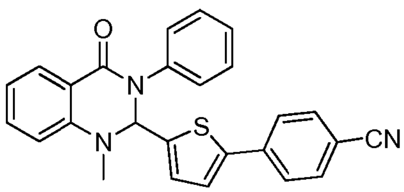 | 4-(5-(1-Methyl-4-oxo-3-phenyl-1,2,3,4-tetrahydroquinazolin-2-yl)thiophen-2-yl) benzonitrile |
| RN-3-012 | 53 | 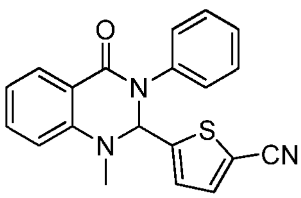 | 5-(1-Methyl-4-oxo-3-phenyl-1,2,3,4-tetrahydroquinazolin-2-yl)thiophene-2-carbonitrile |
| RN-3-066 | 54 | 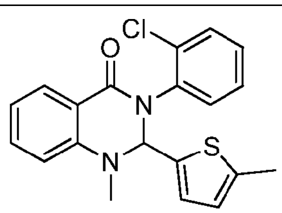 | 3-(2-chlorophenyl)-1-methyl-2-(5-methylthiophen-2-yl)-2,3-dihydroquinazolin-4(1H)-one |

(suite)

| molécule | Composé | structure | Nom |
|---|---|---|---|
| RN-3-067 | 55 | 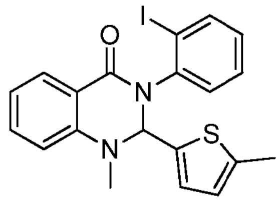 | 3-(2-iodophenyl)-1-methyl-2-(5-methylthiophen-2-yl)-2,3-dihydroquinazolin-4(1H)-one |
| RN-3-068 | 56 | 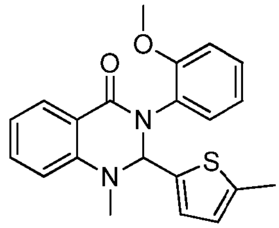 | 3-(2-methoxyphenyl)-1-methyl-2-(5-methylthiophen-2-yl)-2,3-dihydroquinazolin-4(1H)-one |
| RN-3-069 | 57 | 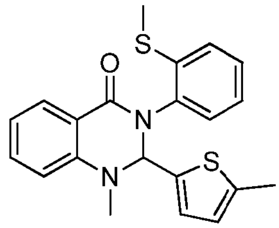 | 1-methyl-3-(2-(methylthio)phenyl)-2-(5-methylthiophen-2-yl)-2,3-dihydroquinazolin-4(1H)-one |
| RN-3-070 | 58 | 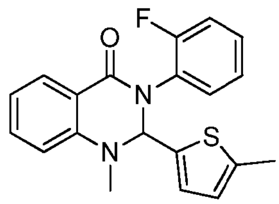 | 3-(2-fluorophenyl)-1-methyl-2-(5-methylthiophen-2-yl)-2,3-dihydroquinazolin-4(1H)-one |
| RN-3-089 | 59 | 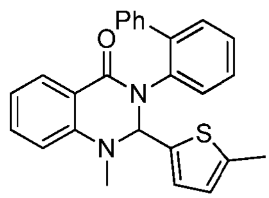 | 3-([1,1'-biphenyl]-2-yl)-1-methyl-2-(5-methylthiophen-2-yl)-2,3-dihydroquinazolin-4(1H)-one |
| RN-3-098 | 60 | 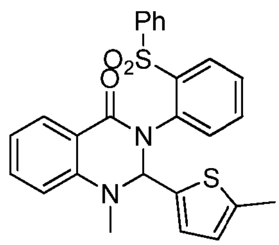 | 1-methyl-2-(5-methylthiophen-2-yl)-3-(2-(phenylsulfonyl)phenyl)-2,3-dihydroquinazolin-4(1H)-one |
| RN-3-122 | 61 | 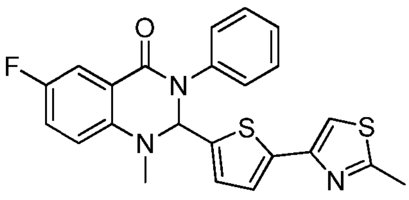 | 6-fluoro-1-methyl-2-(5-(2-methylthiazol-4-yl)thiophen-2-yl)-3-phenyl-2,3-dihydroquinazolin-4(1H)-one |

(suite)

| molécule | Composé | structure | Nom |
|---|---|---|---|
| RN-3-061 | 62 | 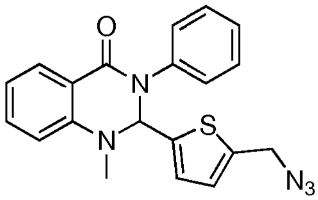 | 2-(5-(azidomethyl)thiophen-2-yl)-1 -methyl-3-phenyl-2,3-dihydroquinazolin-4(1H)-one |
| VP37 | 63 | 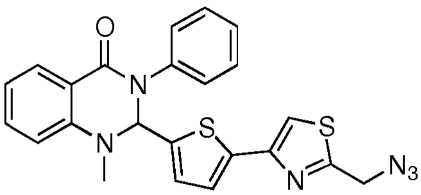 | 2-(5-(2-(azidomethyl)thiazol-4-yl) thiophen-2-yl)-1-methyl-3-phenyl-2,3-dihydroquinazolin-4(1H)-one |
| VP153 | 64 | 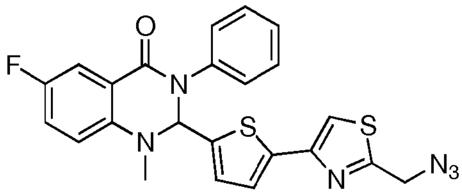 | 2-(5-(2-(azidomethyl)thiazol-4-yl) thiophen-2-yl)-6-fluoro-1-methyl-3-phenyl-2,3-dihydroquinazolin-4 (1H)-one |
| VP22 | 65 | 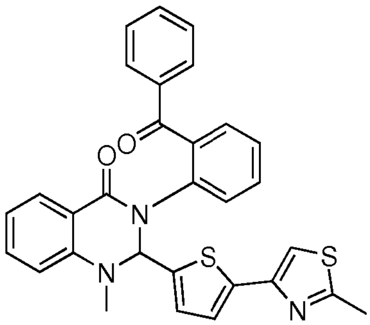 | 3-(2-benzoylphenyl)-1-methyl-2-(5-(2-methylthiazol-4-yl)thiophen-2-yl)-2,3-dihydroquinazolin-4(1H)-one |
| VP104 | 66 | 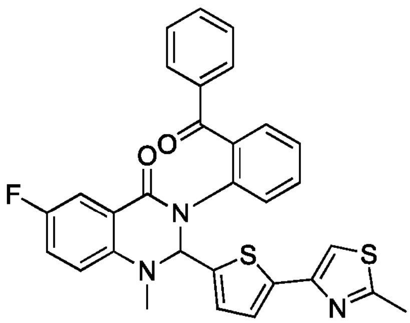 | 3-(2-benzoylphenyl)-6-fluoro-1-methyl-2-(5-(2-methylthiazol-4-yl)thiophen-2-yl)-2,3-dihydroquinazolin-4(1H)-one |
| KH071-4 | 67 | 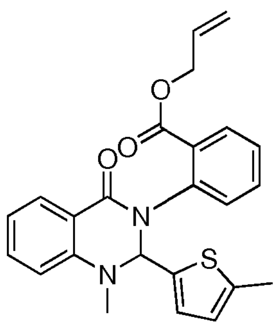 | allyl 2-(1-methyl-2-(5-methylthiophen-2-yl)-4-oxo-1,4-dihydroquinazolin-3 (2H)-yl)benzoate |

(suite)

| molécule | Composé | structure | Nom |
|---|---|---|---|
| KH093-4 | 68 | 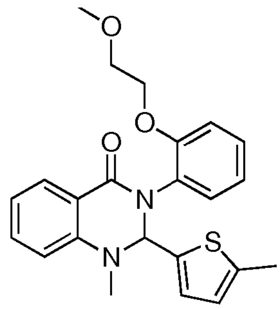 | 3-(2-(2-methoxyethoxy)phenyl)-1-methyl-2-(5-methylthiophen-2-yl)-2,3-dihydroquinazolin-4(1H)-one |
| KH112-2 | 69 | 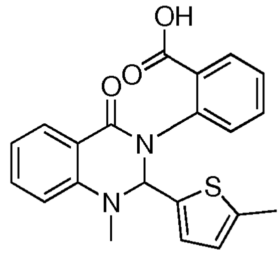 | 2-(1-methyl-2-(5-methylthiophen-2-yl)-4-oxo-1,4-dihydroquinazolin-3(2H)-yl) benzoic acid |

Selon un mode de réalisation préféré de la description, les composés de formule générale (I) sont tels que :

- **$R^1$** est tel que défini précédemment ; c'est-à-dire que $R^1$ représente un atome d'hydrogène, un atome d'halogène ou un radical alcoxy de 1 à 3 atomes de carbone ; de préférence, $R^1$ est un atome d'hydrogène ou un atome d'halogène ; de préférence $R^1$ est lié au carbone C7 du cycle quinazolinone ;
- **$R^2$** est un cycle phényle, éventuellement substitué par : un radical phényle, un atome d'halogène, un groupe $-SO_2$-phényle, un groupe -S-X ou -O-X, X étant un radical alkyle de 1 à 4 atomes de carbone, de préférence un radical méthyle, un alcoxy de 1 à 4 atomes de carbone, un PEG de formule $-(CH_2-CH_2-O)_n-R$ avec n valant 1 à 10, de préférence n vaut 1, et R est un atome d'hydrogène ou un radical méthyle, de préférence R est un radical méthyle, ou un groupe -CO-Y ou -CO-O-Y, Y étant choisi parmi un atome d'hydrogène, un radical alkyle de 1 à 4 atomes de carbone, un cycle phényle ou un groupe allyle ($-CH_2-CH=CH_2$) ;
- **$R^3$** représente un cycle thiophène substitué sur le C5' du thiophène, le cycle quinazolinone étant lié au C2' du thiophène, par :

    * un radical alkyle de 1 à 3 atomes de carbone, préférentiellement méthyle ;
    * un atome d'halogène ;
    * un radical phényle éventuellement substitué par un radical alcoxy de 1 à 3 atomes de carbone, un groupe -CN, un groupe $-NO_2$ ou un groupe -COX ou -COOX avec X un radical alkyle de 1 à 4 atomes de carbone ou une combinaison de ces substituants, de préférence, un radical méthyle ;
    * un groupe -SY, Y étant un radical alkyle de 1 à 4 atomes de carbone ou un radical phényle ;
    * un groupe -CN ;
    * un groupe $-CH_2-N_3$ ;
    * un hétérocycle aromatique de 5 ou 6 atomes qui peut être choisi parmi un cycle furane, thiophène, pyrrole, pyrroline, pyrrolidine, dioxolane, oxazole, thiazole, imidazole, imidazoline, imidazolidine, pyrazole, isoxazole, isothiazole, pyrane, pyridine, pipéridine, dioxane, morpholine, pyridazine, pyrimidine, pyrazine, de préférence, ledit hétérocycle de 5 ou 6 atomes est un thiophène, une pyridine, un furane, un thiazole ; éventuellement substitué par au moins un radical alkyle de 1 à 3 atomes de carbone et/ou un groupe $-CH_2-N_3$ ;

- **$R^4$** est tel que défini précédemment, c'est-à-dire qu'il représente un atome d'hydrogène ou un radical méthyle ;
  à l'exclusion du 3-(4-Chlorophenyl)-2-(5-methylthiophen-2-yl)-2,3-dihydroquinazolin-4(1*H*)-one et du composé tel que $R^1$ est un atome d'hydrogène, $R^2$ un radical phényle, $R^3$ un radical 5-méthylthiophèn-2-yl et $R^4$ un atome d'hydrogène.

La structure chimique qui suit représente la numérotation des carbones utilisée pour définir l'emplacement des

substitutions dans les composés de ce mode de réalisation préféré de la description

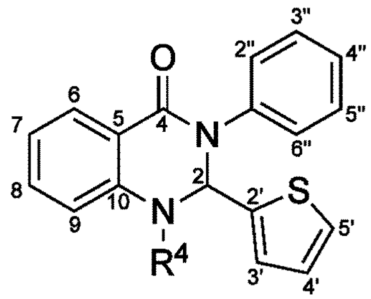

Les composés préférés selon ce mode de réalisation de la description sont ceux choisis parmi les composés 7, 9, 13, 14, 16 à 28, 30 à 69.

Selon une variante préférée du mode de réalisation qui précède, $R^2$ est un cycle phényle substitué sur son carbone C2" (composés 23, 31, 32, 54 à 60 et 65 à 69) et/ou $R^4$ est un radical méthyle (composés 35 à 69).

Selon un autre mode de réalisation préféré de la description, les composés de formule générale (I) sont tels que :

- **$R^1$** est un atome d'hydrogène ou un atome de fluor ;
- **$R^2$** est un cycle phényle éventuellement substitué par : un atome de chlore, d'iode ou de fluor, un radical $-OCH_3$, $-SCH_3$, $-SO_2$-phényle ou un phényle ;
- **$R^3$** est un radical thiophène substitué par un radical méthyle, éthyle, phényle, $-SCH_3$, 2-thiophene, 2-furane, 3-furane, 2-pyridine, 4-(2-Me thiazole), un atome de brome, S-phényle et
- **$R^4$** est un atome d'hydrogène ou un radical méthyle ;

à l'exclusion du composé tel que $R^1$ est un atome d'hydrogène, $R^2$ un radical phényle, $R^3$ un radical 5-méthylthiophèn-2-yl et $R^4$ un atome d'hydrogène,
et leur sels pharmaceutiquement acceptables.

Les composés préférés selon cet autre mode de réalisation de la description sont ceux choisis parmi 7, 9, 13, 17, 18, 19, 20, 21, 25, 31, 32, 34, 35, 36, 37, 38, 39, 40, 41, 42, 43, 44, 47, 54, 55, 56, 57, 58, 59, 60, 61.

Les composés de formule générale (I) pour lesquels $R^4$ est un atome d'hydrogène sont obtenus à partir des composés de type imine analogues de Retro-2 (voir la Demande EP 2 145 873) par cyclisation en milieu basique (voie A) ou directement par réaction de l'anthranilamide avec l'aldéhyde (voie B) :

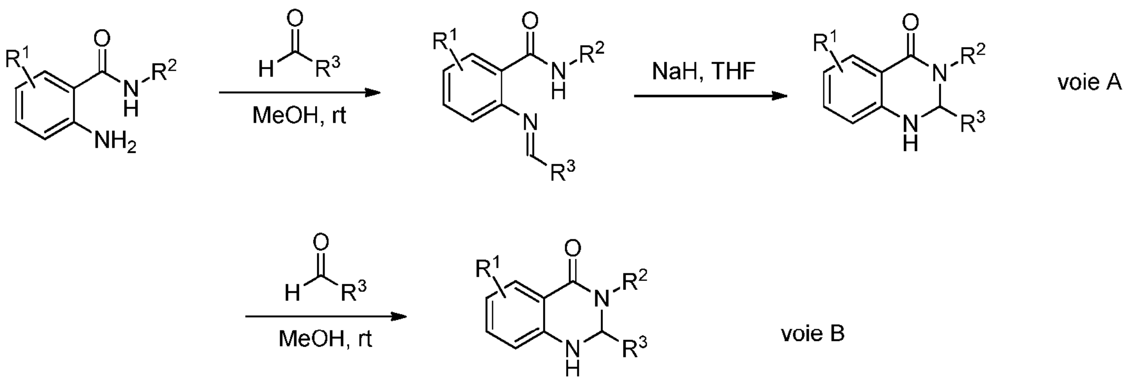


Les composés de formule générale telle que $R^4$ est un substituant méthyle comme représenté ci-contre :

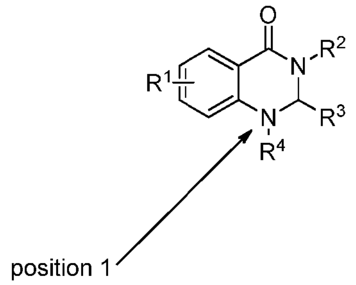


sont obtenus par déprotonation de l'amine en position 1 et piégeage de l'anion par de l'iodure de méthyle.

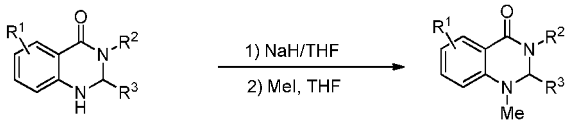


où $R^1$, $R^2$ et $R^3$ sont définis comme précédemment.

Certains des composés selon la description, en particulier, les composés 45, 46, 47, 48, 49, 50, 51 et 52 ont été synthétisés par la voie décrite ci-après :

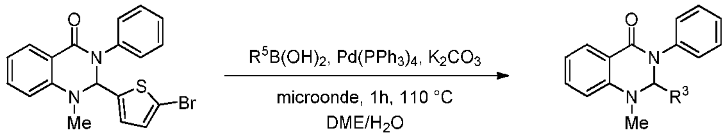


ou par couplage du composé 43 avec PhSH en présence de $Pd_2dba_3$ (0.1 équiv.), dppf (0.15 équiv.) et *t*BuOK (2 équiv.) dans le dioxane, 1h à 140 °C au micro-onde (composé 44) ou $ZnCN_2$ en présence de $Pd_2dba_3$ (0.05 équiv.), dppf (0.1 équiv.) dans le DMF, 1h à 150 °C au micro-onde (composé 53).

Les composés selon la description sont des substances pharmacologiquement actives et trouvent leur intérêt grâce à leur effet inhibiteur des toxines à mode d'action intracellulaire, en particulier, de la ricine.

L'utilisation des composés de formule générale (I), incluant les différents modes de réalisation et leurs variantes préférées, est particulièrement avantageuse pour prévenir et/ou traiter les désordres provoqués par les toxines à mode d'action intracellulaire utilisant le transport rétrograde pour intoxiquer les cellules eucaryotes de mammifères.

Plus spécifiquement, les toxines à mode d'action intracellulaire sont : notamment la ricine (produite dans les graines de la plante *Ricinus communis*), la toxine de Shiga et les toxines Shiga-like (Stxs) produite par *Shigella dysenteriae* (Stx) et *E. coli* (StxI et Stx2), la toxine cholérique (Ctx de *Vibrio cholerae* responsable du choléra), la toxine pertussique (*Bordetella pertussis* agent de la coqueluche), la cytotoxine subtilase et l'entérotoxine thermolabile (*E. coli*),

L'utilisation des composés selon la description s'avère efficace que le sujet ingère ou inhale la toxine, ou encore que la toxine lui soit injectée.

Ainsi les composés selon la description pourront être utilisés pour prévenir et/ou traiter l'intoxication des cellules eucaryotes mammifères par des toxines à mode d'action intracellulaire.

De façon concrète, une toxine comme la ricine, lorsqu'elle est inhalée, conduit à des signes d'irritation oculaire (sensation de brûlure, larmoiement, conjonctivite plus ou moins sévère) et pharyngée ainsi qu'une irritation respiratoire plus ou moins marquée : toux, dyspnée, œdème pulmonaire pouvant conduire à un syndrome de détresse respiratoire aigu (SDRA). Il est à noter qu'il existe un risque de réaction anaphylactique. La dose létale est de 1 mg/kg de poids corporel (Ministère de la santé, France).

Ainsi, la description se rapporte à l'utilisation d'un composé de formule générale (I) pour prévenir et/ou traiter les intoxications à la ricine ou à d'autres toxines à mode d'action intracellulaire et protéger contre ces intoxications les cellules eucaryotes, notamment, épithéliales, oculaires, pharyngiennes, trachéales, bronchiques, cutanées, musculaires, en particulier, des cellules épithéliales pulmonaires et digestives, de préférence intestinales, de mammifères, de préférence de l'homme.

La description se rapporte également à des compositions pharmaceutiques ou médicament comprenant un ou plusieurs composés de formule générale (I) dans un véhicule pharmaceutiquement acceptable.

Par pharmaceutiquement acceptable, on entend compatible avec une administration à un sujet, de préférence un mammifère, par toute voie d'administration.

L'homme du métier saura adapter la formulation des composés de formule générale (I) selon leurs propriétés physico-chimiques et leur voie d'administration.

Le médicament pourra être administré par voie orale (notamment dans la cavité buccale ou en administration sublinguale), parentérale, pulmonaire, oculaire, nasale.... D'autres modes d'administration envisageables comprennent la voie intra-péritonéale (i.p), intraveineuse (i.v.), sous-cutanée (s.c.), intramusculaire (i.m.), transcutanée, transdermale, intracathécale, épidurale, sous-mucosale, par inhalation nasale ou pulmonaire.

Les modes d'administration des composés de formule générale (I) préférés sont ceux utilisant les voies aériennes (inhalation nasale ou pulmonaire), orale (ingestion), parentérale ou locale (topique).

La présente description se rapporte ainsi à des compositions, en particulier des compositions pharmaceutiques, comprenant au moins un composé de formule générale (I) selon la description et, éventuellement, un véhicule pharmaceutiquement acceptable, des excipients stabilisants et des adjuvants classiquement utilisés.

La quantité de composé de formule générale (I) à administrer au mammifère dépend de l'activité propre de ce composé, activité qui peut être mesurée par des moyens qui sont exposés dans les exemples. Cette quantité dépend également de la gravité de la pathologie à traiter, notamment de la quantité de toxine absorbée et de la voie par laquelle elle l'a été, elle dépend enfin de l'âge et du poids de l'individu à traiter.

Outre les dispositions qui précèdent, la divulgation comprend encore d'autres dispositions qui ressortiront de la description qui va suivre, qui se réfèrent à des exemples de mise en œuvre de la présente description, ainsi qu'aux figures annexées dans lesquelles :

La **Figure 1** représente les structures cristallographiques de la ricine (A, dont le nom dans la base de données des structures cristallographiques est pdb 2AAI) et de la Stx2 (B, pdb 1R4P).

La **Figure 2** illustre schématiquement l'entrée et le routage intracellulaire de la ricine (A) et des toxines de Shiga (B).

La **Figure 3** est une représentation schématique du test cellulaire mis en œuvre dans la partie expérimentale.

La **Figure 4** illustre la méthode de détermination de R et des $EC_{50}$ réalisée dans la partie II des exemples.

La **Figure 5** illustre la méthode de calcul de l'$IC_{50}$ réalisée dans la partie II des exemples ; les courbes de toxicité sont réalisées en l'absence d'inhibiteur (Contrôle) puis en présence d'inhibiteur à différentes concentrations.

## Exemples

### I. Synthèse de composés selon la description

Les composés de formule générale (I) qui suivent sont préparés selon le schéma réactionnel qui suit :

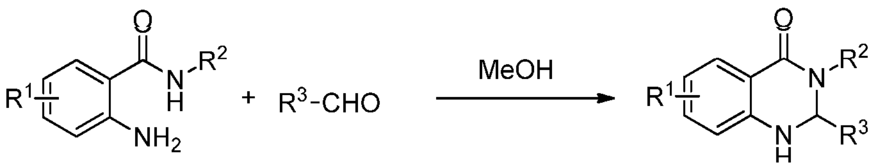


Protocole : dans un tube scellé sont ajoutés le 2-amino benzamide (4.71 mmol), du THF (23 mL, 0.2M) puis l'aldéhyde (4.71 mmol) et de l'acide para-toluène sulfonique (10 mol %, 0.5 mmol). La solution est chauffée à 75 °C et agitée à cette température jusqu'à disparition complète des produits de départ (suivi par chromatographie sur couche mince, typiquement une nuit de temps de réaction). Après refroidissement à température ambiante, de la silice est ajoutée et le THF est évaporé. Une purification par chromatographie sur gel de silice suivie d'une évaporation fournit les produits attendus sous forme de solides. Les composés 1 à 34 ne font pas partie de l'invention.

**Tableau II.** Molécules cycliques de type 2,3-dihydroquinazolin-4(1*H*)-one

| molécule | composé | Nom | rendement | LC/ MS | $^{1}H$ | $^{13}C$ |
|---|---|---|---|---|---|---|
| RN-1-013 | 1 | 2-(3-Methoxyphenyl)-3-phenyl-2,3-dihydroquinazolin-4(1*H*)-one | 88% | x | x | x |
| RN-1-019 | 2 | 2-(4-(Dimethylamino)phenyl)-3-phenyl- 2,3-dihydroquinazolin-4(1*H*)-one | 77% | x | x | x |
| RN-1-021 | 3 | 2,3-Diphenyl-2,3-dihydroquinazolin-4(1*H*)-one | 84% | x | x | x |

(suite)

| molécule | composé | Nom | rendement | LC/MS | $^{1}H$ | $^{13}C$ |
|---|---|---|---|---|---|---|
| RN-1-027 | 4 | 2-(4-Methoxyphenyl)-3-phenyl-2,3-dihydroquinazolin-4(1*H*)-one | 78% | x | x | x |
| RN-1-066 | 5 | 2-(3-Methylthiophen-2-yl)-3-phenyl-2,3-dihydroquinazolin-4(1*H*)-one | 85% | x | x | x |
| RN-1-067 | 6 | 2-(4-Methylthiophen-2-yl)-3-phenyl-2,3-dihydroquinazolin-4(1*H*)-one | 65% | x | x | x |
| RN-1-068 | 7 | 2-(5-Ethylthiophen-2-yl)-3-phenyl-2,3-dihydroquinazolin-4(1*H*)-one | 77% | x | x | x |
| RN-1-086 | 8 | 3-((5R,7S)-3-Hydroxyadamantan-1 -yl)-2-(5-methylthiophen-2-yl)-2,3-dihydroquinazolin-4 (1*H*)-one | 81% | x | x | x |
| RN-1-100 | 9 | 2-(5-Bromothiophen-2-yl)-3-phenyl-2,3-dihydroquinazolin-4(1*H*)-one | 91% | x | x | x |
| RN-1-118 | 10 | 2-(5-Methylthiophen-2-yl)-3-(pyridin-3-yl)-2,3-dihydroquinazolin-4(1*H*)-one | 34% | x | x | x |
| RN-1-120 | 11 | 2-(5-Methylthiophen-2-yl)-3-(pyridin-4-yl)-2,3-dihydroquinazolin-4(1*H*)-one | 64% | x | x | x |
| RN-1-132 | 12 | 2-(5-Methylthiophen-2-yl)-3-(pyrimidin-4-yl)-2,3-dihydroquinazolin-4(1*H*)-one | 53% | x | x | x |
| RN-1-148 | 13 | 3-Phenyl-2-(5-phenylthiophen-2-yl)-2,3-dihydroquinazolin-4(1*H*)-one | 86% | x | x | x |
| RN-1-149 | 14 | 3-([1,1'-Biphenyl]-4-yl)-2-(5-methylthiophen-2-yl)-2,3-dihydroquinazolin-4(1*H*)-one | 73% | x | x | x |
| RN-1-151 | 15 | 2-(5-Methylthiophen-2-yl)-3-(3-(trifluoromethyl) phenyl)-2,3-dihydroquinazolin-4(1*H*)-one | 68% | x | x | x |
| RN-1-162 | 16 | 8-Methyl-2-(5-methylthiophen-2-yl)-3-phenyl-2,3-dihydroquinazolin-4(1*H*)-one | 99% | x | x | x |
| RN-1-173 | 17 | 2-(5-(Methylthio)thiophen-2-yl)-3-phenyl-2,3-dihydroquinazolin-4(1*H*)-one | 82% | x | x | - |
| RN-1-174 | 18 | 2-([2,2'-Bithiophen]-5-yl)-3-phenyl-2,3-dihydroquinazolin-4(1*H*)-one | 86% | x | x | x |
| RN-1-175 | 19 | 3-Phenyl-2-(5-(pyridin-2-yl)thiophen-2-yl)-2,3-dihydroquinazolin-4(1*H*)-one | 72% | x | x | x |
| RN-1-176 | 20 | 2-(5-(Furan-2-yl)thiophen-2-yl)-3-phenyl-2,3-dihydroquinazolin-4(1*H*)-one | 64% | x | x | x |
| RN-1-177 | 21 | 2-(5-(2-Methylthiazol-4-yl)thiophen-2-yl)-3-phenyl-2,3-dihydroquinazolin-4(1*H*)-one | 68% | x | x | x |
| RN-2-120 | 22 | 3-(4-Fluorophenyl)-2-(5-methylthiophen-2-yl)-2,3-dihydroquinazolin-4(1*H*)-one | 80% | x | x | x |
| RN-2-137 | 23 | 3-(2-Fluorophenyl)-2-(5-methylthiophen-2-yl)-2,3-dihydroquinazolin-4(1*H*)-one | 93% | x | x | x |
| RN-2-161 | 24 | 6-Methoxy-2-(5-methylthiophen-2-yl)-3-phenyl-2,3-dihydroquinazolin-4(1*H*)-one | 89% | x | x | x |

(suite)

| molécule | composé | Nom | rendement | LC/ MS | $^1H$ | $^{13}C$ |
|---|---|---|---|---|---|---|
| RN-2-162 | 25 | 6-Fluoro-2-(5-methylthiophen-2-yl)-3-phenyl-2,3-dihydroquinazolin-4(1*H*)-one | 70% | x | x | x |
| RN-2-163 | 26 | 7-Fluoro-2-(5-methylthiophen-2-yl)-3-phenyl-2,3-dihydroquinazolin-4(1*H*)-one | 80% | x | x | x |
| RN-2-177 | 27 | 3-(4-Bromophenyl)-2-(5-methylthiophen-2-yl)-2,3-dihydroquinazolin-4(1*H*)-one | 80% | x | x | x |
| RN-2-178 | 28 | 3-(3-Bromophenyl)-2-(5-methylthiophen-2-yl)-2,3-dihydroquinazolin-4(1*H*)-one | 91% | x | x | x |
| RN-2-180 | 29 | 3-(4-Chlorophenyl)-2-(5-methylthiophen-2-yl)-2,3-dihydroquinazolin-4(1*H*)-one | 89% | x | x | x |
| RN-2-181 | 30 | 3-(3-Chlorophenyl)-2-(5-methylthiophen-2-yl)-2,3-dihydroquinazolin-4(1*H*)-one | 91% | x | x | x |
| RN-2-183 | 31 | 3-(2-(Methylthio)phenyl)-2-(5-methylthiophen-2-yl)-2,3-dihydroquinazolin-4(1*H*)-one | 79% | x | x | x |
| RN-2-182 | 32 | 3-(2-Chlorophenyl)-2-(5-methylthiophen-2-yl)-2,3-dihydroquinazolin-4(1*H*)-one | 92% | x | x | x |
| RN-2-199 | 33 | 6-Iodo-2-(5-methylthiophen-2-yl)-3-phenyl-2,3-dihydroquinazolin-4(1*H*)-one | 92% | | x | |
| RN-3-121 | 34 | 6-fluoro-2-(5-(2-methylthiazol-4-yl)thiophen-2-yl)-3-phenyl-2,3-dihydroquinazolin-4(1H)-one | 36% | x | x | x |

Synthèse des composés de formule générale (I) pour lesquels $R^4$ est un radical méthyle :

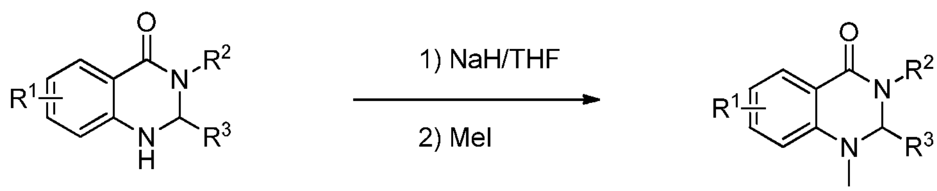


Protocole : à une suspension de NaH (1.5 équiv.) dans du tétrahydrofuranne anhydre (0.3M) est ajouté goutte à goutte une solution de N-H tetrahydroquinazolin-4-ones (1 équiv.) dans du THF anhydre (0.5M) à 0°C. Après 30min, de l'iodométhane (1.1 équiv.) est ajouté goutte à goutte. Après 10 minutes de réaction, la solution est réchauffée à température ambiante et le milieu réactionnel est agité pendant 3h. La réaction est stoppée par ajout d'une solution saturée de $NaHCO_3$. La phase aqueuse est extraite par du $CH_2Cl_2$ (3 x 50 mL). les phases organiques sont réunies puis lavées par une solution 1M solution d'HCl (2 x 10 mL), séchées sur $Na_2SO_4$ anhydre, filtrée et concentrée à l'évaporateur rotatif. Une purification par chromatographie sur gel de silice fournit les produits attendus sous forme de solides.

*Tableau III*

| molécule | composé | nom | rendement | LC/MS | $^1H$ | $^{13}C$ |
|---|---|---|---|---|---|---|
| RN-1-181 | 35 | 1 -Methyl-2-(5-methylthiophen-2-yl)-3-phenyl-2,3-dihydroquinazolin-4(1*H*)-one | 85% à partir de RN-1-001 | x | x | x |
| RN-1-186 | 36 | 1-Methyl-3-phenyl-2-(5-phenylthiophen-2-yl)-2,3-dihydroquinazolin-4(1*H*)-one | 69% à partir de RN-1-069 | x | x | x |

(suite)

| molécule | composé | nom | rendement | LC/MS | $^{1}H$ | $^{13}C$ |
|---|---|---|---|---|---|---|
| RN-1-192 | 37 | 1-Methyl-3-phenyl-2-(5-(pyridin-2-yl)thiophen-2-yl)-2,3-dihydroquinazolin-4(1 *H*)-one | 97% à partir de RN-1-101 | x | x | x |
| RN-1-196 | 38 | 1-Methyl-2-(5-(methylthio)thiophen-2-yl)-3-phenyl- 2,3-dihydroquinazolin-4(1*H*)-one | 91% à partir de RN-1-077 | x | x | x |
| RN-1-197 | 39 | 2-([2,2'-Bithiophen]-5-yl)-1-methyl-3-phenyl-2,3-dihydroquinazolin-4(1 *H*)-one | 95% à partir de RN-1-080 | x | x | x |
| RN-2-001 | 40 | 2-(5-(Furan-2-yl)thiophen-2-yl)-1-methyl-3-phenyl-2,3-dihydroquinazolin-4(1 *H*)-one | 97% à partir de RN-1-104 | x | x | x |
| RN-2-005 | 41 | 2-(5-Ethylthiophen-2-yl)-1-methyl-3-phenyl-2,3-dihydroquinazolin-4(1 *H*)-one | 84% à partir de RN-1-068 | x | x | x |
| RN-2-015 | 42 | 1-Methyl-2-(5-(2-methylthiazol-4-yl)thiophen-2-yl)-3-phenyl-2,3-dihydroquinazolin-4(1*H*)-one | 83% à partir de RN-1-105 | x | x | x |
| RN-2-016 | 43 | 2-(5-Bromothiophen-2-yl)-1-methyl- 3 -phenyl- 2,3-dihydroquinazolin-4(1*H*)-one | 85% à partir de RN-1-100 | x | x | x |
| RN-2-019 | 44 | 1-Methyl-3-phenyl-2-(5-(phenylthio)thiophen-2-yl)-2,3-dihydroquinazolin-4(1*H*)-one | 91% à partir de RN-2-016 | x | x | x |
| RN-2-029 | 45 | 2-(5-(3,4-Dimethoxyphenyl)thiophen-2-yl)-1-methyl-3-phenyl-2,3-dihydroquinazolin-4(1*H*)-one | 88% à partir de RN-2-016 | x | x | x |
| RN-2-032 | 46 | 1-Methyl-2-(5-(3-nitrophenyl)thiophen-2-yl)-3-phenyl-2,3-dihydroquinazolin-4(1*H*)-one | 90% à partir de RN-2-016 | x | x | x |
| RN-2-034 | 47 | 2-(5-(Furan-3-yl)thiophen-2-yl)-1-methyl-3-phenyl-2,3-dihydroquinazolin-4(1*H*)-one | 80% à partir de RN-2-016 | x | x | x |
| RN-2-049 | 48 | Methyl 4-(5-(1-methyl-4-oxo-3-phenyl-1,2,3,4-tetrahydroquinazolin-2-yl)thiophen-2-yl)benzoate | 80% à partir de RN-2-016 | x | x | x |
| RN-2-050 | 49 | 2-(5-(4-Acetylphenyl)thiophen-2-yl)-1-methyl-3-phenyl-2,3-dihydroquinazolin-4(1*H*)-one | 89% à partir de RN-2-016 | x | x | x |
| RN-2-053 | 50 | 1-Methyl-3-phenyl-2-(5-(3,4,5-trimethoxyphenyl) thiophen-2-yl)-2,3-dihydroquinazolin-4(1H)-one | 73% à partir de RN-2-016 | x | x | x |
| RN-2-057 | 51 | 1-methyl-2-(5-(4-methyl-3-nitrophenyl)thiophen-2-yl)-3-phenyl-2,3-dihydroquinazolin-4(1H)-one | 76% à partir de RN-2-016 | x | x | x |

(suite)

| molécule | composé | nom | rendement | LC/MS | $^{1}H$ | $^{13}C$ |
|---|---|---|---|---|---|---|
| RN-2-059 | 52 | 4-(5-(1-Methyl-4-oxo-3-phenyl-1,2,3,4-tetrahydroquinazolin-2-yl)thiophen-2-yl) benzonitrile | 78% à partir de RN-2-016 | x | x | x |
| RN-3-012 | 53 | 5-(1-Methyl-4-oxo-3-phenyl-1,2,3,4-tetrahydroquinazolin-2-yl)thiophene-2-carbonitrile | 68% à partir de RN-2-016 | x | | |
| RN-3-066 | 54 | 3-(2-chlorophenyl)-1-methyl-2-(5-methylthiophen-2-yl)-2,3-dihydroquinazolin-4(1H)-one | 67% | x | x | x |
| RN-3-067 | 55 | 3-(2-iodophenyl)-1-methyl-2-(5-methylthiophen-2-yl)-2,3-dihydroquinazolin-4(1H)-one | 46% | x | x | x |
| RN-3-068 | 56 | 3-(2-methoxyphenyl)-1-methyl-2-(5-methylthiophen-2-yl)-2,3-dihydroquinazolin-4 (1H)-one | 28% | x | x | x |
| RN-3-069 | 57 | 1-methyl-3-(2-(methylthio)phenyl)-2-(5-methylthiophen-2-yl)-2,3-dihydroquinazolin-4 (1H)-one | 73% | x | x | x |
| RN-3-070 | 58 | 3-(2-fluorophenyl)-1-methyl-2-(5-methylthiophen-2-yl)-2,3-dihydroquinazolin-4(1H)-one | 51% | x | x | X |
| RN-3-089 | 59 | 3-([1,1'-biphenyl]-2-yl)-1-methyl-2-(5-methylthiophen-2-yl)-2,3-dihydroquinazolin-4 (1H)-one | 74% | x | x | X |
| RN-3-098 | 60 | 1-methyl-2-(5-methylthiophen-2-yl)-3-(2-(phenylsulfonyl)phenyl)-2,3-dihydroquinazolin-4(1H)-one | 65% | x | x | X |
| RN-3-122 | 61 | 6-fluoro-1-methyl-2-(5-(2-methylthiazol-4-yl) thiophen-2-yl)-3-phenyl-2,3-dihydroquinazolin-4 (1H)-one | 71% | x | x | X |

## II. Mesure de l'activité protectrice des composés vis-à-vis des toxines sur cellules

### *II.A. Protocole expérimental*

Les composés ont été testés soit sur cellules A549 (cellules épithéliales pulmonaires humaines) soit sur cellules HeLa (cellules de cancer utérin humain), respectivement contre la ricine et contre les toxines de Shiga (Stx-1 et/ou Stx-2). Les cellules humaines sont cultivées à 37°C dans une atmosphère contenant 5% de $CO_2$ sur des flasques de culture de 150 $cm^2$ dans du milieu DMEM (Dulbecco's Modified Eagle Medium) contenant 100 U/mL de pénicilline et 100 $\mu$g/mL de streptomycine. Les cellules sont ensemencées à une densité de 50000 cellules par puits dans des plaques 96 puits à fond en scintillant solide Cytostar-T (**Figure 3**). Les cellules (100 $\mu$L dans du DMEM complet: DMEM + 10% de sérum de veau fœtal, SVF) sont pré-incubées ou non avec les inhibiteurs (50 $\mu$L; différentes concentrations, pré-incubation de 3 h). Le milieu complet complémenté de toxine (50 $\mu$L, gamme de concentration variable) est ensuite ajouté à chaque puits. Après une incubation de 20 h, le milieu (200 $\mu$L) est éliminé et remplacé par un milieu DMEM sans leucine (Eurobio) contenant 10% de SVF et 0,5 $\mu$Ci/mL de $^{14}C$-leucine (GE). Après une incubation de 7 h à 37°C, l'incorporation de radioactivité par les cellules est déterminée par lecture des plaques par un compteur à scintillation Wallac 1450 Microbeta trilux (PE).

Comme ces toxines bloquent la synthèse des protéines, les cellules affectées ne sont plus capables d'incorporer la leucine radiomarquée. Par contre, les cellules traitées par des inhibiteurs synthétisent toujours des protéines et incorporent donc l'acide aminé radiomarqué. Comme les cellules concentrent le radioélément suffisamment près du fond du puits, cela entraine une excitation du scintillant contenu dans les plaques et conduit à l'émission de photons détectée par le compteur à scintillation (mesure en coups par minutes, cpm). Ces données sont ensuite exprimées en pourcentage de synthèse de protéine par les cellules. Les courbes de cytotoxicité peuvent ainsi être tracées (R, **Figure**

**3**) sans inhibiteur (ronds blancs) ou en présence d'un inhibiteur (ronds noirs). L'analyse des données par régression non linéaire permet d'estimer l'$EC_{50}$, soit la concentration efficace pour laquelle on observe 50 % d'assimilation de leucine radioactive ce qui correspond à 50% de cellules viables. Plus la valeur de l'$EC_{50}$ est élevée, plus la protection cellulaire est importante car il faut alors une concentration plus élevée de toxine pour générer la même cytotoxicité. Il est ainsi possible de déterminer l'efficacité des inhibiteurs en calculant le ratio des $EC_{50}$ (R, *cf.* **Figure 3**). Plus la valeur est élevée (> 1), plus la protection des cellules est importante.

***Résultats***

Le Tableau IV ci-dessous présente les résultats sous la forme d'un indice de protection (ratio $EC_{50}$ composé / $EC_{50}$ ricine) : plus il est élevé, plus les cellules sont protégées contre l'action de la ricine (effet protecteur si > 1). Les composés 7, 9, 13, 17 à 21, 25, 31 à 32 et 34 ne font pas partie de l'invention.

**Tableau IV.** Evaluation des activités biologiques des composés selon la description.

| molécule | composé | R (ricine) | R (Stx) |
|---|---|---|---|
| Contrôle | Retro- 2 | 2,7 | 25 |
| RN-1-068 | 7 | 2,6 | 120 |
| RN-1-100 | 9 | 1,8 | 27,9 |
| RN-1-148 | 13 | 4,2 | 153 |
| RN-1-173 | 17 | 2,8 | 43 |
| RN-1-174 | 18 | 5,6 | 50 |
| RN-1-175 | 19 | 3,1 | 46 |
| RN-1-176 | 20 | 0,7 | 41 |
| RN-1-177 | 21 | 7,3 | 109 |
| RN-2-162 | 25 | - | 133 |
| RN-2-183 | 31 | - | 99 |
| RN-2-182 | 32 | - | 68 |
| RN-3-121 | 34 | - | 295 |
| RN-1-181 | 35 | 6,4 | 80 |
| RN-1-186 | 36 | 6,1 | 29 |
| RN-1-192 | 37 | 4,3 | 111 |
| RN-1-196 | 38 | 4,6 | 60 |
| RN-1-197 | 39 | 4,0 | 36 |
| RN-2-001 | 40 | - | 27 |
| RN-2-005 | 41 | 6,7 | 173 |
| RN-2-015 | 42 | 8,1 | 85 |
| RN-2-016 | 43 | 4,6 | 40 |
| RN-2-019 | 44 | 3,6 | 71 |
| RN-2-034 | 47 | 4,1 | 49 |
| RN-3-066 | 54 | - | 27 |
| RN-3-067 | 55 | - | 25 |
| RN-3-068 | 56 | - | 26 |
| RN-3-069 | 57 | - | 25 |
| RN-3-070 | 58 | - | 25 |

(suite)

| molécule | composé | R (ricine) | R (Stx) |
|---|---|---|---|
| RN-3-089 | 59 | - | 27 |
| RN-3-098 | 60 | - | 320 |
| RN-3-122 | 61 | - | 40 |

Ces essais démontrent que les composés selon la description testés présentent une meilleure protection contre la ricine et/ou Stx que Retro-2 cyclisé.

***II.B. Méthode alternative d'évaluation de l'activité des composés selon l la description contre la toxine Shiga***

Le Tableau IV ci-dessus présente la mesure du pouvoir inhibiteur des composés sous la forme d'un indice de protection R (ratio $EC_{50}$ toxine + composé / $EC_{50}$ toxine ; *cf.* **Figure 4**). Plus la valeur de R est élevée, plus les cellules sont protégées contre l'action de la toxine. Pour les composés présentant des indices R proches de celui de Retro-2 (R proche de 25 dans le cas de la toxine de Shiga), des données complémentaires plus précises sont fournies (Tableau V complémentaire ci-après) pour s'affranchir notamment de la variabilité de R.

En effet la valeur de R pour une molécule donnée varie selon les expériences. Ce n'est pas une valeur absolue et R ne peut pas être utilisée pour comparer les différentes molécules entre elles, sauf si elles étaient testées toutes le même jour dans une même expérience. Cette valeur permet seulement de comparer l'activité d'un composé vis-à-vis du contrôle (i.e. Retro-2) et de déterminer si la molécule est plus active ou non que Retro-2 à une concentration donnée. En effet, les résultats du Tableau IV sont obtenus à partir d'expériences réalisées avec une seule et unique concentration de composé (30 $\mu$M).

Les résultats du Tableau V complémentaire sont basés sur l'étude d'une gamme de concentration de chacun des composés testés et sont plus précis car ils permettent de s'affranchir de la valeur de R et de fournir une valeur $IC_{50}$ comparable entre tous les composés. $IC_{50}$ correspond à la concentration de composé donnant 50% de son pouvoir inhibiteur. Plus cette concentration est faible, plus l'inhibiteur est puissant.

***Protocole et calcul de $IC_{50}$***

Le test sur plaque 96-puits est le même que celui décrit dans la partie II.A. ci-avant. Une plaque 96 puits entière est nécessaire pour calculer l'$IC_{50}$ de chaque composé.

Six courbes de cytototoxicité sont obtenues en l'absence puis en présence de concentrations croissantes d'inhibiteur. Pour chaque concentration (C) d'inhibiteur, un pourcentage de protection est déterminé à partir de la valeur de R calculée par le logiciel Prism avec Rmax correspondant à la valeur maximale de R de la série:

$$\%\ protection = [(R-1)/(Rmax-1)] * 100$$

L'$IC_{50}$ est ensuite calculée, à l'aide du logiciel Prism par régression non linéaire, à partir d'un graphique où sont reportés pour chaque concentration de composé le pourcentage de protection cellulaire correspondant (*cf.* **Figure 5**). Le composé 9 ne fait pas partie de l'invention.

***Résultats***

*Tableau V*

| Molécule | Composé | $IC_{50}$($\mu$M) |
|---|---|---|
| Contrôle | Retro- 2 | 27.3 |
| RN-1-100 | 9 | 13.7 |
| RN-2-001 | 40 | 1.5 |
| RN-3-066 | 54 | 8.2 |

(suite)

| Molécule | Composé | $IC_{50}$(μM) |
|---|---|---|
| RN-3-067 | 55 | 5.4 |
| RN-3-068 | 56 | 5.4 |
| RN-3-069 | 57 | 3.8 |
| RN-3-070 | 58 | 5.5 |
| RN-3-089 | 59 | 3.7 |
| RN-3-061 | 62 | 12 |
| VP37 | 63 | 3-5 |
| VP153 | 64 | <3 |
| VP22 | 65 | 1-3 |
| VP104 | 66 | 1 |
| KH071-4 | 67 | 13 |
| KH093-4 | 68 | 4.2 |
| KH112-2 | 69 | 11.2 |

CONCLUSION

Ces résultats montrent que les composés testés sont tous des inhibiteurs de l'effet cellulaire de la toxine Shiga plus puissants que le composé Retro-2.

**III. Mise en évidence du blocage du transport rétrograde**

***Protocole expérimental***

Le transport rétrograde de la sous-unité B de la toxine de Shiga (StxB) à l'appareil de Golgi a été quantifié par un test de sulfatation décrit en détail dans la littérature (Amessou M. et al. Curr. Protoc. Cell. Biol. 2006, Chapter 15: Unit 15.10). Le principe est le suivant : un variant de StxB appelé StxB-$Sulf_2$, qui porte un tandem de sites de reconnaissance de sulfatation protéique, est internalisé en présence de $^{35}SO_4^{2-}$. Une fois que StxB-$Sulf_2$ atteint l'appareil de Golgi, les sulfo-transferases localisées dans l'appareil de Golgi catalysent le transfert du sulfate radioactif sur StxB-$Sulf_2$. Après lyse cellulaire, immuno-précipitation et électrophorèse sur gel, le [$^{35}S$]-StxB-$Sulf_2$ peut être détecté et quantifié par autoradiographie. Dans une publication précédente (Stechmann B. et al. Cell 2010, 141, 231-24), il a été montré que le transport rétrograde des cellules traitées avec Retro-1 ou Retro-2 était diminué de 90% dans certaines conditions expérimentales.

***Résultats***

Le Tableau VI indique pour les cellules traitées ou non avec différentes molécules, la mesure du transport rétrograde en pourcentage. 100% indique aucun effet sur le transport rétrograde, 0% indique un blocage complet de ce transport. Les composés 7, 13 et 17 à 21 ne font pas partie de l'invention.

***Tableau VI.*** *Evaluation du transport rétrograde des cellules en présence de composés selon la description.*

| molécule | Composé | % contrôle |
|---|---|---|
| DMSO | | 100 |
| Retro 2 | | 36,9 |
| RN-1-068 | 7 | 13,5 |
| RN-1-148 | 13 | 13,1 |
| RN-1-173 | 17 | 22,8 |

(suite)

| molécule | Composé | % contrôle |
|---|---|---|
| RN-1-174 | 18 | 17,1 |
| RN-1-175 | 19 | 10,7 |
| RN-1-176 | 20 | 6,5 |
| RN-1-177 | 21 | 0,0 |
| RN-1-186 | 36 | 7,1 |
| RN-1-192 | 37 | 10,3 |
| RN-1-196 | 38 | 18,2 |
| RN-1-197 | 39 | 6,2 |
| RN-2-001 | 40 | 5,3 |
| RN-2-005 | 41 | 0,0 |
| RN-2-015 | 42 | 0,0 |

CONCLUSION

Les composés ci-dessus montrent une activité marquée de blocage du transport rétrograde, supérieure à celle de Retro-2, qui peut s'expliquer soit par une bonne affinité pour la cible et/ou une bonne internalisation dans la cellule soit par une bonne stabilité de ces composés en milieu biologique.

**Revendications**

**1.** Composé de formule générale (I) :

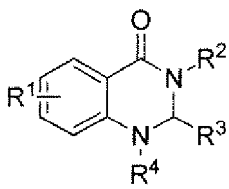

(I)

où

- **$R^1$** représente un atome d'hydrogène, un atome d'halogène ou un radical alcoxy de 1 à 3 atomes de carbone ;
- **$R^2$** est un cycle phényle, éventuellement substitué par : un radical phényle, un atome d'halogène, un groupe $-SO_2$-phényle, un groupe -S-X ou O-X, X étant un radical alkyle de 1 à 4 atomes de carbone, un alcoxy de 1 à 4 atomes de carbone, un PEG de formule $-(CH_2-CH_2-O)_n-R$ avec n valant 1 à 10 et R est un atome d'hydrogène ou un radical méthyle, ou un groupe -CO-Y ou -CO-O-Y, Y étant choisi parmi un atome d'hydrogène, un radical alkyle de 1 à 4 atomes de carbone, un cycle phényle ou un groupe allyle ($-CH_2-CH-CH_2$) ;
- **$R^3$** représente un cycle thiophène substitué par :

  * un radical alkyle de 1 à 3 atomes de carbone ;
  * un atome d'halogène ;
  * un radical phényle éventuellement substitué par un radical alcoxy de 1 à 3 atomes de carbone, un groupe -CN, un groupe $-NO_2$ ou un groupe -COX ou -COOX avec X un radical alkyle de 1 à 4 atomes de carbone ou une combinaison de ces substituants ;
  * un groupe -SY, Y étant un radical alkyle de 1 à 4 atomes de carbone ou un radical phényle ;
  * un groupe -CN ;
  * un groupe $-CH_2-N_3$ ;

* un hétérocycle aromatique de 5 ou 6 atomes qui peut être choisi parmi un cycle furane, thiophène, pyrrole, pyrroline, pyrrolidine, dioxolane, oxazole, thiazole, imidazole, imidazoline, imidazolidine, pyrazole, isoxazole, isothiazole, pyrane, pyridine, pipéridine, dioxane, morpholine, pyridazine, pyrimidine, pyrazine ; éventuellement substitué par au moins un radical alkyle de 1 à 3 atomes de carbone et/ou un groupe $-CH_2-N_3$ ;

- $\mathbf{R^4}$ représente un radical méthyle.

**2.** Composé de formule générale (I) selon la revendication 1, **caractérisé en ce qu'**il est choisi parmi :

| molécule | composé | Nom |
|---|---|---|
| RN-1-181 | 35 | 1-Methyl-2-(5-methylthiophen-2-yl)-3-phenyl-2,3-dihydroquinazolin-4(1*H*)-one |
| RN-1-186 | 36 | 1-Methyl-3-phenyl-2-(5-phenylthiophen-2-yl)-2,3-dihydroquinazolin-4(1*H*)-one |
| RN-1-192 | 37 | 1-Methyl-3-phenyl-2-(5-(pyridin-2-yl)thiophen-2-yl)-2,3-dihydroquinazolin-4( 1 *H*)-one |
| RN-1-196 | 38 | 1-Methyl-2-(5-(methylthio)thiophen-2-yl)-3-phenyl-2,3-dihydroquinazolin-4(1*H*)-one |
| RN-1-197 | 39 | 2-([2,2'-Bithiophen]-5-yl)-1-methyl-3-phenyl-2,3-dihydroquinazolin-4( 1*H*)-one |
| RN-2-001 | 40 | 2-(5-(Furan-2-yl)thiophen-2-yl)-1-methyl-3-phenyl-2,3-dihydroquinazolin-4(1 *H*)-one |
| RN-2-005 | 41 | 2-(5-Ethylthiophen-2-yl)-1-methyl-3-phenyl-2,3-dihydroquinazolin-4(1*H*)-one |
| RN-2-015 | 42 | 1-Methyl-2-(5-(2-methylthiazol-4-yl)thiophen-2-yl)-3-phenyl-2,3-dihydroquinazolin-4 (1*H*)-one |
| RN-2-016 | 43 | 2-(5-Bromothiophen-2-yl)-1-methyl-3-phenyl-2,3-dihydroquinazolin-4(1*H*)-one |
| RN-2-019 | 44 | 1-Methyl-3-phenyl-2-(5-(phenylthio)thiophen-2-yl)-2,3-dihydroquinazolin-4( 1*H*)-one |
| RN-2-029 | 45 | 2-(5-(3,4-Dimethoxyphenyl)thiophen-2-yl)-1-methyl-3-phenyl-2,3-dihydroquinazolin-4 (1*H*)-one |
| RN-2-032 | 46 | 1-Methyl-2-(5-(3-nitrophenyl)thiophen-2-yl)-3-phenyl-2,3-dihydroquinazolin-4(1*H*)-one |
| RN-2-034 | 47 | 2-(5-(Furan-3-yl)thiophen-2-yl)-1-methyl-3-phenyl-2,3-dihydroquinazolin-4(1*H*)-one |
| RN-2-049 | 48 | Methyl 4-(5-(1-methyl-4-oxo-3-phenyl-1,2,3,4-tetrahydroquinazolin-2-yl)thiophen-2-yl) benzoate |
| RN-2-050 | 49 | 2-(5-(4-Acetylphenyl)thiophen-2-yl)-1-methyl-3-phenyl-2,3-dihydroquinazolin-4( 1*H*)-one |
| RN-2-053 | 50 | 1-Methyl-3-phenyl-2-(5-(3,4,5-trimethoxyphenyl)thiophen-2-yl)-2,3-dihydroquinazolin-4 (1*H*)-one |
| RN-2-057 | 51 | 1-methyl-2-(5-(4-methyl-3-nitrophenyl)thiophen-2-yl)-3-phenyl-2,3-dihydroquinazolin-4(1 H)-one |
| RN-2-059 | 52 | 4-(5-(1-Methyl-4-oxo-3-phenyl-1,2,3,4-tetrahydroquinazolin-2-yl)thiophen-2-yl) benzonitrile |
| RN-3-012 | 53 | 5-(1-Methyl-4-oxo-3-phenyl-1,2,3,4-tetrahydroquinazolin-2-yl)thiophene-2-carbonitrile |
| RN-3-066 | 54 | 3-(2-chlorophenyl)-1-methyl-2-(5-methylthiophen-2-yl)-2,3-dihydroquinazolin-4(1H)-one |
| RN-3-067 | 55 | 3-(2-iodophenyl)-1-methyl-2-(5-methylthiophen-2-yl)-2,3-dihydroquinazolin-4( 1 H)-one |
| RN-3-068 | 56 | 3-(2-methoxyphenyl)-1-methyl-2-(5-methylthiophen-2-yl)-2,3-dihydroquinazolin-4(1 H)-one |
| RN-3-069 | 57 | 1-methyl-3-(2-(methylthio)phenyl)-2-(5-methylthiophen-2-yl)-2,3-dihydroquinazolin-4( 1 H)-one |
| RN-3-070 | 58 | 3-(2-fluorophenyl)-1-methyl-2-(5-methylthiophen-2-yl)-2,3-dihydroquinazolin-4(1 H)-one |
| RN-3-089 | 59 | 3-([1, 1'-biphenyl]-2-yl)-1 -methyl-2-(5-methylthiophen-2-yl)-2,3-dihydroquinazolin-4( 1 H)-one |

(suite)

| molécule | composé | Nom |
|---|---|---|
| RN-3-098 | 60 | 1-methyl-2-(5-methylthiophen-2-yl)-3-(2-(phenylsulfonyl)phenyl)-2,3-dihydroqumazolin-4 (1H)-one |
| RN-3-122 | 61 | 6-fluoro-1-methyl-2-(5-(2-methylthiazol-4-yl)thiophen-2-yl)-3-phenyl-2,3-dihydroquinazolin-4(1H)-one |
| RN-3-061 | 62 | 2-(5-(azidomethyl)thiophen-2-yl)-1-methyl-3-phenyl-2,3-dihydroquinazolin-4(1H)-one |
| VP37 | 63 | 2-(5-(2-(azidomethyl)thiazol-4-yl)thiophen-2-yl)-1-methyl-3-phenyl-2,3-dihydroquinazolin-4(1H)-one |
| VP153 | 64 | 2-(5-(2-(azidomethyl)thiazol-4-yl)thiophen-2-yl)-6-fluoro-1-methyl-3-phenyl-2,3-dihydroquinazolin-4(1H)-one |
| VP22 | 65 | 3-(2-benzoylphenyl)-1-methyl-2-(5-(2-methylthiazol-4-yl)thiophen-2-yl)-2,3-dihydroquinazolin-4(1H)-one |
| VP104 | 66 | 3-(2-benzoylphenyl)-6-fluoro-1-methyl-2-(5-(2-methylthiazol-4-yl)thiophen-2-yl)-2,3-dihydroquinazolin-4(1H)-one |
| KH071-4 | 67 | allyl 2-(1-methyl-2-(5-methylthiophen-2-yl)-4-oxo-1,4-dihydroquinazolin-3(2H)-yl) benzoate |
| KH093-4 | 68 | 3-(2-(2-methoxyethoxy)phenyl)-1-methyl-2-(5-methylthiophen-2-yl)-2,3-dihydroquinazolin-4(1H)-one |
| KH112-2 | 69 | 2-(1-methyl-2-(5-methylthiophen-2-yl)-4-oxo-1,4-dihydroquinazolin-3(2H)-yl)benzoic acid |

**3.** Composé de formule générale (I) selon la revendication 1, **caractérisé en ce que** $R^2$ est un cycle phényle substitué sur son carbone C2".

**4.** Composé de formule générale (I) selon la revendication 3, **caractérisé en ce qu'**il est choisi parmi les composés 54 à 60 et 65 à 69.

**5.** Composé de formule générale (I)

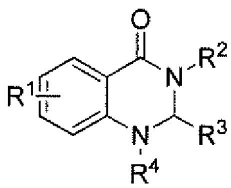

(I)

où

- **$R^1$** représente un atome d'hydrogène, un atome d'halogène ou un radical alcoxy de 1 à 3 atomes de carbone ;
- **$R^2$** est un cycle phényle, éventuellement substitué par : un radical phényle, un atome d'halogène, un groupe $-SO_2$-phényle, un groupe -S-X ou -O-X, X étant un radical alkyle de 1 à 4 atomes de carbone, alcoxy de 1 à 4 atomes de carbone ; un PEG de formule $-(CH_2-CH_2-O)_n-R$ avec n valant 1 à 10 et R représente un atome d'hydrogène ou un radical méthyle ; ou un groupe -CO-Y ou -CO-O-Y, Y étant choisi parmi un atome d'hydrogène, un radical alkyle de 1 à 4 atomes de carbone, un cycle phényle ou un groupe allyle ($-CH_2-CH=CH_2$) ;
- $R^3$ représente un cycle thiophène substitué par :

  * un radical alkyle de 1 à 3 atomes de carbone ;
  * un atome d'halogène ;
  * un radical phényle éventuellement substitué par un radical alcoxy de 1 à 3 atomes de carbone, un groupe -CN, un groupe $-NO_2$ ou un groupe -COX ou -COOX avec X un radical alkyle de 1 à 4 atomes de carbone ou une combinaison de ces substituants ;

* un groupe -SY, Y étant un radical alkyle de 1 à 4 atomes de carbone ou un radical phényle ;
* un groupe -CN ;
* un groupe $-CH_2-N_3$ ;
* un hétérocycle aromatique de 5 ou 6 atomes qui peut être choisi parmi un cycle furane, thiophène, pyrrole, pyrroline, pyrrolidine, dioxolane, oxazole, thiazole, imidazole, imidazoline, imidazolidine, pyrazole, isoxazole, isothiazole, pyrane, pyridine, pipéridine, dioxane, morpholine, pyridazine, pyrimidine, pyrazine ; éventuellement substitué par au moins un radical alkyle de 1 à 3 atomes de carbone et/ou un groupe $-CH_2-N_3$ ;

- **R⁴** représente un radical méthyle

pour son utilisation pour la prévention et/ou le traitement des désordres induits par les toxines à mode d'action intracellulaire utilisant le transport rétrograde.

6. Composé de formule générale (I) pour son utilisation selon la revendication 5, **caractérisé en ce que** $R^2$ est un cycle phényle substitué sur son carbone C2".

7. Composé de formule générale (I) selon l'une quelconque des revendications 5 ou 6 pour son utilisation selon la revendication 5, **caractérisé en ce que** lesdites toxines à mode d'action intracellulaire sont choisies parmi la ricine, la toxine de Shiga et les toxines Shiga-like (Stxs) produite par *Shigella dysenteriae* (Stx) et *E. coli* (Stx1 et Stx2), la toxine cholérique (Ctx de *Vibrio cholerae* responsable du choléra), la toxine pertussique (*Bordetella pertussis* agent de la coqueluche), la cytotoxine subtilase et l'entérotoxine thermolabile (*E. coli*).

8. Composition pharmaceutique ou médicament comprenant au moins un composé de formule générale (I) tels que définis dans l'une quelconque des revendications 1 à 4, dans un véhicule pharmaceutiquement acceptable, **caractérisé en ce que** ladite composition pharmaceutique ou ledit médicament est adapté à une administration par les voies aérienne, orale, parentérale ou locale.

9. Composition pharmaceutique ou médicament comprenant au moins un composé de formule générale (I) tels que définis dans l'une quelconque des revendications 1 à 4 pour une utilisation selon la revendication 5 ou la revendication 7, dans un véhicule pharmaceutiquement acceptable, **caractérisé en ce que** ladite composition pharmaceutique ou ledit médicament est adapté à une administration par les voies aérienne, orale, parentérale ou locale.

**Patentansprüche**

1. Verbindung der allgemeinen Formel (I):

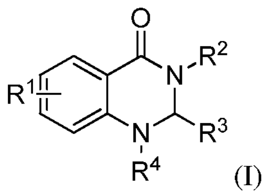

(I)

wobei

- **R¹** ein Wasserstoffatom, ein Halogenatom oder einen Alkoxyrest mit 1 bis 3 Kohlenstoffatomen darstellt;
- **R²** ein Phenylring ist, gegebenenfalls substituiert durch: einen Phenylrest, ein Halogenatom, eine $-SO_2$-Phenylgruppe, eine -S-X- oder -O-X-Gruppe, wobei X ein Alkylrest mit 1 bis 4 Kohlenstoffatomen, ein Alkoxy mit 1 bis 4 Kohlenstoffatomen, ein PEG der Formel $-(CH_2-CH_2-O)_n-R$ ist, mit n im Bereich von 1 bis 10 und R ein Wasserstoffatom oder ein Methylrest ist, oder eine -CO-Y- oder -CO-O-Y-Gruppe, wobei Y ausgewählt ist aus einem Wasserstoffatom, einem Alkylrest mit 1 bis 4 Kohlenstoffatomen, einem Phenylring oder einer Allylgruppe ($-CH_2-CH=CH_2$);
- **R³** einen Thiophenring darstellt, substituiert durch:

* einen Alkylrest mit 1 bis 3 Kohlenstoffatomen;
* ein Halogenatom;

* einen Phenylrest, gegebenenfalls substituiert durch einen Alkoxyrest mit 1 bis 3 Kohlenstoffatomen, eine -CN-Gruppe, eine $-NO_2$-Gruppe oder eine -COX- oder-COOX-Gruppe, mit X ein Alkylrest mit 1 bis 4 Kohlenstoffatomen, oder eine Kombination dieser Substituenten;
* eine -SY-Gruppe, wobei Y ein Alkylrest mit 1 bis 4 Kohlenstoffatomen oder ein Phenylrest ist;
* eine -CN-Gruppe;
* eine $-CH_2-N_3$-Gruppe;
* einen aromatischen Heterocyclus mit 5 oder 6 Atomen, der ausgewählt sein kann aus einem Furan-, Thiophen-, Pyrrol-, Pyrrolin-, Pyrrolidin-, Dioxolan-, Oxazol-, Thiazol-, Imidazol-, Imidazolin-, Imidazolidin-, Pyrazol-, Isoxazol-, Isothiazol-, Pyran-, Pyridin-, Piperidin-, Dioxan-, Morpholin-, Pyridazin-, Pyrimidin-, Pyrazinring; gegebenenfalls substituiert durch mindestens einen Alkylrest mit 1 bis 3 Kohlenstoffatomen und/oder eine $-CH_2-N_3$-Gruppe;

- **$R^4$** einen Methylrest darstellt.

**2.** Verbindung der allgemeinen Formel (I) nach Anspruch 1, **dadurch gekennzeichnet, dass** sie ausgewählt ist aus:

| Molekül | Verbindung | Name |
|---|---|---|
| RN-1-181 | 35 | 1-Methyl-2-(5-methylthiophen-2-yl)-3-phenyl-2,3-dihydrochinazolin-4(1*H*)-on |
| RN-1-186 | 36 | 1-Methyl-3-phenyl-2-(5-phenylthiophen-2-yl)-2,3-dihydrochinazolin-4(1*H*)-on |
| RN-1-192 | 37 | 1-Methyl-3-phenyl-2-(5-(pyridin-2-yl)thiophen-2-yl)-2,3-dihydrochinazolin-4(1*H*)-on |
| RN-1-196 | 38 | 1-Methyl-2-(5-(methylthio)thiophen-2-yl)-3-phenyl-2,3-dihydrochinazolin-4(1*H*)-on |
| RN-1-197 | 39 | 2-([2,2'-Bithiophen]-5-yl)-1-methyl-3-phenyl-2,3-dihydrochinazolin-4(1*H*)-on |
| RN-2-001 | 40 | 2-(5-(Furan-2-yl)thiophen-2-yl)-1-methyl-3-phenyl-2,3-dihydrochinazolin-4(1*H*)-on |
| RN-2-005 | 41 | 2-(5-Ethylthiophen-2-yl)-1-methyl-3-phenyl-2,3-dihydrochinazolin-4(1*H*)-on |
| RN-2-015 | 42 | 1-Methyl-2-(5-(2-methylthiazol-4-yl)thiophen-2-yl)-3-phenyl-2,3-dihydrochinazolin-4 (1*H*)-on |
| RN-2-016 | 43 | 2-(5-Bromthiophen-2-yl)-1-methyl-3-phenyl-2,3-dihydrochinazolin-4(1*H*)-on |
| RN-2-019 | 44 | 1-Methyl-3-phenyl-2-(5-(phenylthio)thiophen-2-yl)-2,3-dihydrochinazolin-4(1*H*)-on |
| RN-2-029 | 45 | 2-(5-(3,4-Dimethoxyphenyl)thiophen-2-yl)-1-methyl-3-phenyl-2,3-dihydrochinazolin-4(1*H*)-on |
| RN-2-032 | 46 | 1-Methyl-2-(5-(3-nitrophenyl)thiophen-2-yl)-3-phenyl-2,3-dihydrochinazolin-4 (1*H*)-on |
| RN-2-034 | 47 | 2-(5-(Furan-3-yl)thiophen-2-yl)-1-methyl-3-phenyl-2,3-dihydrochinazolin-4(1*H*)-on |
| RN-2-049 | 48 | Methyl-4-(5-(1-methyl-4-oxo-3-phenyl-1,2,3,4-tetrahydrochinazolin-2-yl)thiophen-2-yl)benzoat |
| RN-2-050 | 49 | 2-(5-(4-Acetylphenyl)thiophen-2-yl)-1-methyl-3-phenyl-2,3-dihydrochinazolin-4 (1*H*)-on |
| RN-2-053 | 50 | 1-Methyl-3-phenyl-2-(5-(3,4,5-trimethoxyphenyl)thiophen-2-yl)-2,3-dihydrochinazolin-4(1*H*)-on |
| RN-2-057 | 51 | 1-Methyl-2-(5-(4-methyl-3-nitrophenyl)thiophen-2-yl)-3-phenyl-2,3-dihydrochinazolin-4(1 H)-on |
| RN-2-059 | 52 | 4-(5-(1-Methyl-4-oxo-3-phenyl-1,2,3,4-tetrahydrochinazolin-2-yl)thiophen-2-yl) benzonitril |
| RN-3-012 | 53 | 5-(1-Methyl-4-oxo-3-phenyl-1,2,3,4-tetrahydrochinazolin-2-yl)thiophen-2-carbonitril |
| RN-3-066 | 54 | 3-(2-Chlorphenyl)-1-methyl-2-(5-methylthiophen-2-yl)-2,3-dihydrochinazolin-4(1 H)-on |
| RN-3-067 | 55 | 3-(2-Iodphenyl)-1-methyl-2-(5-methylthiophen-2-yl)-2,3-dihydrochinazolin-4(1 H)-on |

(fortgesetzt)

| Molekül | Verbindung | Name |
|---|---|---|
| RN-3-068 | 56 | 3-(2-Methoxyphenyl)-1-methyl-2-(5-methylthiophen-2-yl)-2,3-dihydrochinazolin-4(1 H)-on |
| RN-3-069 | 57 | 1-Methyl-3-(2-(methylthio)phenyl)-2-(5-methylthiophen-2-yl)-2,3-dihydrochinazolin-4(1 H)-on |
| RN-3-070 | 58 | 3-(2-Fluorphenyl)-1-methyl-2-(5-methylthiophen-2-yl)-2,3-dihydrochinazolin-4(1 H)-on |
| RN-3-089 | 59 | 3-([1,1'-Biphenyl]-2-yl)-1-methyl-2-(5-methylthiophen-2-yl)-2,3-dihydrochinazolin-4 (1 H)-on |
| RN-3-098 | 60 | 1-Methyl-2-(5-methylthiophen-2-yl)-3-(2-(phenylsulfonyl)phenyl)-2,3-dihydrochinazolin-4(1H)-on |
| RN-3-122 | 61 | 6-Fluor-1-methyl-2-(5-(2-methylthiazol-4-yl)thiophen-2-yl)-3-phenyl-2,3-dihydrochinazolin-4(1 H)-on |
| RN-3-061 | 62 | 2-(5-(Azidomethyl)thiophen-2-yl)-1-methyl-3-phenyl-2,3-dihydrochinazolin-4(1 H)-on |
| VP37 | 63 | 2-(5-(2-(Azidomethyl)thiazol-4-yl)thiophen-2-yl)-1-methyl-3-phenyl-2,3-dihydrochinazolin-4(1 H)-on |
| VP153 | 64 | 2-(5-(2-(Azidomethyl)thiazol-4-yl)thiophen-2-yl)-6-fluor-1-methyl-3-phenyl-2,3-dihydrochinazolin-4(1 H)-on |
| VP22 | 65 | 3-(2-Benzoylphenyl)-1-methyl-2-(5-(2-methylthiazol-4-yl)thiophen-2-yl)-2,3-dihydrochinazolin-4(1 H)-on |
| VP104 | 66 | 3-(2-Benzoylphenyl)-6-fluor-1-methyl-2-(5-(2-methylthiazol-4-yl)thiophen-2-yl)-2,3-dihydrochinazolin-4(1 H)-on |
| KH071-4 | 67 | Allyl-2-(1 -methyl-2-(5-methylthiophen-2-yl)-4-oxo-1,4-dihydrochinazolin-3(2H)-yl) benzoat |
| KH093-4 | 68 | 3-(2-(2-Methoxyethoxy)phenyl)-1-methyl-2-(5-methylthiophen-2-yl)-2,3-dihydrochinazolin-4(1 H)-on |
| KH112-2 | 69 | 2-(1 -Methyl-2-(5-methylthiophen-2-yl)-4-oxo-1,4-dihydrochinazolin-3(2H)-yl) benzoesäure |

**3.** Verbindung der allgemeinen Formel (I) nach Anspruch 1, **dadurch gekennzeichnet, dass** $R^2$ ein an seinem C2"-Kohlenstoff substituierter Phenylring ist.

**4.** Verbindung der allgemeinen Formel (I) nach Anspruch 3, **dadurch gekennzeichnet, dass** sie ausgewählt ist aus den Verbindungen 54 bis 60 und 65 bis 69.

**5.** Verbindung der allgemeinen Formel (I)

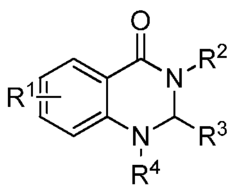

(I)

wobei

- $\mathbf{R^1}$ ein Wasserstoffatom, ein Halogenatom oder einen Alkoxyrest mit 1 bis 3 Kohlenstoffatomen darstellt;

- **$R^2$** ein Phenylring ist, gegebenenfalls substituiert durch: einen Phenylrest, ein Halogenatom, eine -$SO_2$-Phenylgruppe, eine -S-X- oder -O-X-Gruppe, wobei X ein Alkylrest mit 1 bis 4 Kohlenstoffatomen, Alkoxy mit 1 bis 4 Kohlenstoffatomen; ein PEG der Formel $-(CH_2-CH_2-O)_n-R$ ist, mit n im Bereich von 1 bis 10 und R ein Wasserstoffatom oder einen Methylrest darstellt; oder eine -CO-Y- oder -CO-O-Y-Gruppe, wobei Y ausgewählt ist aus einem Wasserstoffatom, einem Alkylrest mit 1 bis 4 Kohlenstoffatomen, einem Phenylring oder einer Allylgruppe ($-CH_2-CH=CH_2$);
- **$R^3$** einen Thiophenring darstellt, substituiert durch:

  * einen Alkylrest mit 1 bis 3 Kohlenstoffatomen;
  * ein Halogenatom;
  * einen Phenylrest, gegebenenfalls substituiert durch einen Alkoxyrest mit 1 bis 3 Kohlenstoffatomen, eine -CN-Gruppe, eine -$NO_2$-Gruppe oder eine -COX- oder -COOX-Gruppe, mit X ein Alkylrest mit 1 bis 4 Kohlenstoffatomen, oder eine Kombination dieser Substituenten;
  * eine -SY-Gruppe, wobei Y ein Alkylrest mit 1 bis 4 Kohlenstoffatomen oder ein Phenylrest ist;
  * eine -CN-Gruppe;
  * eine $-CH_2-N_3$-Gruppe;
  * einen aromatischen Heterocyclus mit 5 oder 6 Atomen, der ausgewählt sein kann aus einem Furan-, Thiophen-, Pyrrol-, Pyrrolin-, Pyrrolidin-, Dioxolan-, Oxazol-, Thiazol-, Imidazol-, Imidazolin-, Imidazolidin-, Pyrazol-, Isoxazol-, Isothiazol-, Pyran-, Pyridin-, Piperidin-, Dioxan-, Morpholin-, Pyridazin-, Pyrimidin-, Pyrazinring; gegebenenfalls substituiert durch mindestens einen Alkylrest mit 1 bis 3 Kohlenstoffatomen und/oder eine $-CH_2-N_3$-Gruppe;

- **$R^4$** einen Methylrest darstellt

für ihre Verwendung zur Prävention und/oder Behandlung von durch Toxine mit intrazellulärer Wirkungsweise, die den retrograden Transport nutzen, induzierten Störungen.

**6.** Verbindung der allgemeinen Formel (I) für ihre Verwendung nach Anspruch 5, **dadurch gekennzeichnet, dass** $R^2$ ein an seinem C2"-Kohlenstoff substituierter Phenylring ist.

**7.** Verbindung der allgemeinen Formel (I) nach einem der Ansprüche 5 oder 6 für ihre Verwendung nach Anspruch 5, **dadurch gekennzeichnet, dass** die Toxine mit intrazellulärer Wirkungsweise ausgewählt sind aus Rizin, Shiga-Toxin und shigaähnlichen Toxinen (Stxs) produziert von *Shigella dysenteriae* (Stx) und *E. coli* (Stx1 und Stx2), Cholera-Toxin (Ctx von *Vibrio cholerae,* Verursacher von Cholera), Pertussis-Toxin (*Bordetella pertussis,* Erreger des Keuchhustens), Subtilase-Zytotoxin und thermolabilem Enterotoxin (*E. coli*).

**8.** Pharmazeutische Zusammensetzung oder Medikament, umfassend mindestens eine Verbindung der allgemeinen Formel (I), wie in einem der Ansprüche 1 bis 4 definiert, in einem pharmazeutisch annehmbaren Träger, **dadurch gekennzeichnet, dass** die pharmazeutische Zusammensetzung oder das Medikament an eine Verabreichung über die Luftwege, orale, parenterale oder lokale Wege angepasst ist.

**9.** Pharmazeutische Zusammensetzung oder Medikament, umfassend mindestens eine Verbindung der allgemeinen Formel (I), wie in einem der Ansprüche 1 bis 4 definiert, für eine Verwendung nach Anspruch 5 oder Anspruch 7, in einem pharmazeutisch annehmbaren Träger, **dadurch gekennzeichnet, dass** die pharmazeutische Zusammensetzung oder das Medikament an eine Verabreichung über die Luftwege, orale, parenterale oder lokale Wege angepasst ist.

## Claims

**1.** Compound of general formula (I):

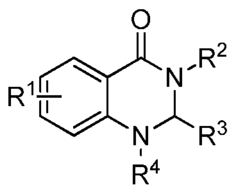

(I)

where

- **$R^1$** represents a hydrogen atom, a halogen atom or an alkoxy radical of 1 to 3 carbon atoms;
- **$R^2$** is a phenyl ring, optionally substituted by: a phenyl radical, a halogen atom, a $-SO_2$-phenyl group, a -S-X or -O-X group, X being an alkyl radical of 1 to 4 carbon atoms, an alkoxy of 1 to 4 carbon atoms, a PEG of formula $-(CH_2-CH_2-O)_n-R$ with n being 1 to 10 and R is a hydrogen atom or a methyl radical, or a -CO-Y or -CO-O-Y group, Y being chosen from a hydrogen atom, an alkyl radical of 1 to 4 carbon atoms, a phenyl ring or an allyl group ($-CH_2-CH=CH_2$);
- **$R^3$** represents a thiophene ring substituted by:

    * an alkyl radical of 1 to 3 carbon atoms;
    * a halogen atom;
    * a phenyl radical optionally substituted by an alkoxy radical of 1 to 3 carbon atoms, a -CN group, a $-NO_2$ group or a -COX or -COOX group with X an alkyl radical of 1 to 4 carbon atoms or a combination of these substituents;
    * a -SY group, Y being an alkyl radical of 1 to 4 carbon atoms or a phenyl radical;
    * a-CN group;
    * a $-CH_2-N_3$ group;
    * an aromatic heterocycle of 5 or 6 atoms which can be chosen from a furane, thiophene, pyrrole, pyrroline, pyrrolidine, dioxolane, oxazole, thiazole, imidazole, imidazoline, imidazolidine, pyrazole, isoxazole, isothiazole, pyran, pyridine, piperidine, dioxane, morpholine, pyridazine, pyrimidine, pyrazine ring; optionally substituted by at least one alkyl radical of 1 to 3 carbon atoms and/or a $-CH_2-N_3$ group;

- **$R^4$** represents a methyl radical.

2. Compound of general formula (I) according to claim 1, **characterised in that** it is chosen from:

| molecule | compound | Name |
|---|---|---|
| RN-1-181 | 35 | 1-Methyl-2-(5-methylthiophen-2-yl)-3-phenyl-2,3-dihydroquinazolin-4(1*H*)-one |
| RN-1-186 | 36 | 1-Methyl-3-phenyl-2-(5-phenylthiophen-2-yl)-2,3-dihydroquinazolin-4(1*H*)-one |
| RN-1-192 | 37 | 1-Methyl-3-phenyl-2-(5-(pyridin-2-yl)thiophen-2-yl)-2,3-dihydroquinazolin-4(1*H*)-one |
| RN-1-196 | 38 | 1-Methyl-2-(5-(methylthio)thiophen-2-yl)-3-phenyl-2,3-dihydroquinazolin-4(1*H*)-one |
| RN-1-197 | 39 | 2-([2,2'-Bithiophen]-5-yl)-1-methyl-3-phenyl-2,3-dihydroquinazolin-4(1*H*)-one |
| RN-2-001 | 40 | 2-(5-(Furan-2-yl)thiophen-2-yl)-1-methyl-3-phenyl-2,3-dihydroquinazolin-4(1*H*)-one |
| RN-2-005 | 41 | 2-(5-Ethylthiophen-2-yl)-1-methyl-3-phenyl-2,3-dihydroquinazolin-4(1*H*)-one |
| RN-2-015 | 42 | 1-Methyl-2-(5-(2-methylthiazol-4-yl)thiophen-2-yl)-3-phenyl-2,3-dihydroquinazolin-4 (1*H*)-one |
| RN-2-016 | 43 | 2-(5-Bromothiophen-2-yl)-1-methyl-3-phenyl-2,3-dihydroquinazolin-4(1*H*)-one |
| RN-2-019 | 44 | 1-Methyl-3-phenyl-2-(5-(phenylthio)thiophen-2-yl)-2,3-dihydroquinazolin-4(1*H*)-one |
| RN-2-029 | 45 | 2-(5-(3,4-Dimethoxyphenyl)thiophen-2-yl)-1-methyl-3-phenyl-2,3-dihydroquinazolin-4 (1*H*)-one |
| RN-2-032 | 46 | 1-Methyl-2-(5-(3-nitrophenyl)thiophen-2-yl)-3-phenyl-2,3-dihydroquinazolin-4(1*H*)-one |

(continued)

| molecule | compound | Name |
|---|---|---|
| RN-2-034 | 47 | 2-(5-(Furan-3-yl)thiophen-2-yl)-1-methyl-3-phenyl-2,3-dihydroquinazolin-4(1*H*)-one |
| RN-2-049 | 48 | Methyl 4-(5-(1-methyl-4-oxo-3-phenyl-1,2,3,4-tetrahydroquinazolin-2-yl)thiophen-2-yl) benzoate |
| RN-2-050 | 49 | 2-(5-(4-Acetylphenyl)thiophen-2-yl)-1-methyl-3-phenyl-2,3-dihydroquinazolin-4(1*H*)-one |
| RN-2-053 | 50 | 1-Methyl-3-phenyl-2-(5-(3,4,5-trimethoxyphenyl)thiophen-2-yl)-2,3-dihydroquinazolin-4 (1*H*)-one |
| RN-2-057 | 51 | 1-methyl-2-(5-(4-methyl-3-nitrophenyl)thiophen-2-yl)-3-phenyl-2,3-dihydroquinazolin-4 (1 H)-one |
| RN-2-059 | 52 | 4-(5-(1-Methyl-4-oxo-3-phenyl-1,2,3,4-tetrahydroquinazolin-2-yl)thiophen-2-yl) benzonitrile |
| RN-3-012 | 53 | 5-(1-Methyl-4-oxo-3-phenyl-1,2,3,4-tetrahydroquinazolin-2-yl)thiophene-2-carbonitrile |
| RN-3-066 | 54 | 3-(2-chlorophenyl)-1-methyl-2-(5-methylthiophen-2-yl)-2,3-dihydroquinazolin-4(1 H)-one |
| RN-3-067 | 55 | 3-(2-iodophenyl)-1-methyl-2-(5-methylthiophen-2-yl)-2,3-dihydroquinazolin-4(1 H)-one |
| RN-3-068 | 56 | 3-(2-methoxyphenyl)-1-methyl-2-(5-methylthiophen-2-yl)-2,3-dihydroquinazolin-4(1 H)-one |
| RN-3-069 | 57 | 1-methyl-3-(2-(methylthio)phenyl)-2-(5-methylthiophen-2-yl)-2,3-dihydroquinazolin-4(1 H)-one |
| RN-3-070 | 58 | 3-(2-fluorophenyl)-1-methyl-2-(5-methylthiophen-2-yl)-2,3-dihydroquinazolin-4(1 H)-one |
| RN-3-089 | 59 | 3-([1,1'-biphenyl]-2-yl)-1-methyl-2-(5-methylthiophen-2-yl)-2,3-dihydroquinazolin-4 (1H)-one |
| RN-3-098 | 60 | 1-methyl-2-(5-methylthiophen-2-yl)-3-(2-(phenylsulfonyl)phenyl)-2,3-dihydroquinazolin-4(1 H)-one |
| RN-3-122 | 61 | 6-fluoro-1-methyl-2-(5-(2-methylthiazol-4-yl)thiophen-2-yl)-3-phenyl-2,3-dihydroquinazolin-4(1 H)-one |
| RN-3-061 | 62 | 2-(5-(azidomethyl)thiophen-2-yl)-1-methyl-3-phenyl-2,3-dihydroquinazolin-4(1 H)-one |
| VP37 | 63 | 2-(5-(2-(azidomethyl)thiazol-4-yl)thiophen-2-yl)-1-methyl-3-phenyl-2,3-dihydroquinazolin-4(1H)-one |
| VP153 | 64 | 2-(5-(2-(azidomethyl)thiazol-4-yl)thiophen-2-yl)-6-fluoro-1-methyl-3-phenyl-2,3-dihydroquinazolin-4(1 H)-one |
| VP22 | 65 | 3-(2-benzoylphenyl)-1-methyl-2-(5-(2-methylthiazol-4-yl)thiophen-2-yl)-2,3-dihydroquinazolin-4(1 H)-one |
| VP104 | 66 | 3-(2-benzoylphenyl)-6-fluoro-1-methyl-2-(5-(2-methylthiazol-4-yl)th iophen-2-yl)-2,3-dihydroquinazolin-4(1 H)-one |
| KH071-4 | 67 | allyl 2-(1-methyl-2-(5-methylthiophen-2-yl)-4-oxo-1,4-dihydroquinazolin-3(2H)-yl) benzoate |
| KH093-4 | 68 | 3-(2-(2-methoxyethoxy)phenyl)-1-methyl-2-(5-methylthiophen-2-yl)-2,3-dihydroquinazolin-4(1 H)-one |
| KH112-2 | 69 | 2-(1-methyl-2-(5-methylthiophen-2-yl)-4-oxo-1,4-dihydroquinazolin-3(2H)-yl)benzoic acid |

3. Compound of general formula (I) according to claim 1, **characterised in that** $R^2$ is a phenyl ring substituted on its carbon C2".

4. Compound of general formula (I) according to claim 3, **characterised in that** it is chosen from the compounds 54

to 60 and 65 to 69.

**5.** Compound of general formula (I)

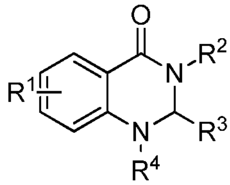

(I)

where

- **$R^1$** represents a hydrogen atom, a halogen atom or an alkoxy radical of 1 to 3 carbon atoms;
- **$R^2$** is a phenyl ring, optionally substituted by: a phenyl radical, a halogen atom, a $-SO_2$-phenyl group, a -S-X or -O-X group, X being an alkyl radical of 1 to 4 carbon atoms, alkoxy of 1 to 4 carbon atoms; a PEG of formula $-(CH_2-CH_2-O)_n-R$ with n equaling 1 to 10 and R represents a hydrogen atom or a methyl radical; or a -CO-Y or - CO-O-Y group, Y being chosen from a hydrogen atom, an alkyl radical of 1 to 4 carbon atoms, a phenyl ring or an allyl group ($-CH_2-CH=CH_2$);
- **$R^3$** represents a thiophene ring substituted by:

  * an alkyl radical of 1 to 3 carbon atoms;
  * a halogen atom;
  * a phenyl radical optionally substituted by an alkoxy radical of 1 to 3 carbon atoms, a -CN group, a $-NO_2$ group or a -COX or -COOX group with X an alkyl radical of 1 to 4 carbon atoms or a combination of these substituents;
  * a -SY group, Y being an alkyl radical of 1 to 4 carbon atoms or a phenyl radical;
  * a-CN group;
  * a $-CH_2-N_3$ group;
  * an aromatic heterocycle of 5 or 6 atoms which can be chosen from a furane, thiophene, pyrrole, pyrroline, pyrrolidine, dioxolane, oxazole, thiazole, imidazole, imidazoline, imidazolidine, pyrazole, isoxazole, isothiazole, pyran, pyridine, piperidine, dioxane, morpholine, pyridazine, pyrimidine, pyrazine ring; optionally substituted by at least one alkyl radical of 1 to 3 carbon atoms and/or a $-CH_2-N_3$ group;

- **$R^4$** represents a methyl radical

for its use for preventing and/or treating disorders induced by toxins with intracellular activity using retrograde transport.

**6.** Compound of general formula (I) for its use according to claim 5, **characterised in that** $R^2$ is a phenyl ring substituted on its carbon C2".

**7.** Compound of general formula (I) according to any one of claims 5 or 6 for its use according to claim 5, **characterised in that** said toxins with intracellular activity are chosen from ricin, Shiga toxin and Shiga-like toxins (Stxs) produced by *Shigella dysenteriae* (Stx) and E. coli (Stx1 and Stx2), choleric toxin (Ctx of *Vibrio cholerae* responsible for cholera), pertussis toxin (*Bordetella pertussis* agent), subtilase cytotoxin and thermolabile enterotoxin (*E. coli*).

**8.** Pharmaceutical composition or medicament comprising at least one compound of general formula (I) such as defined in any one of claims 1 to 4, in a pharmaceutically acceptable vehicle, **characterised in that** said pharmaceutical composition or said medicament is adapted to the aerial, oral, parenteral or local pathways administration.

**9.** Pharmaceutical composition or medicament comprising at least one compound of general formula (I) such as defined in any one of claims 1 to 4 for a use according to claim 5 or claim 7, in a pharmaceutically acceptable vehicle, **characterised in that** said pharmaceutical composition or said medicament is adapted to the aerial, oral, parenteral or local pathways administration.

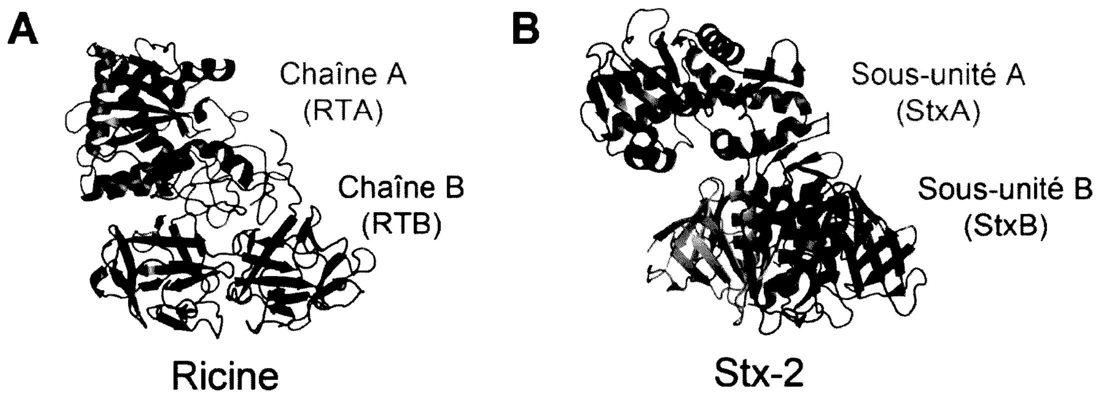


**Figure 1**

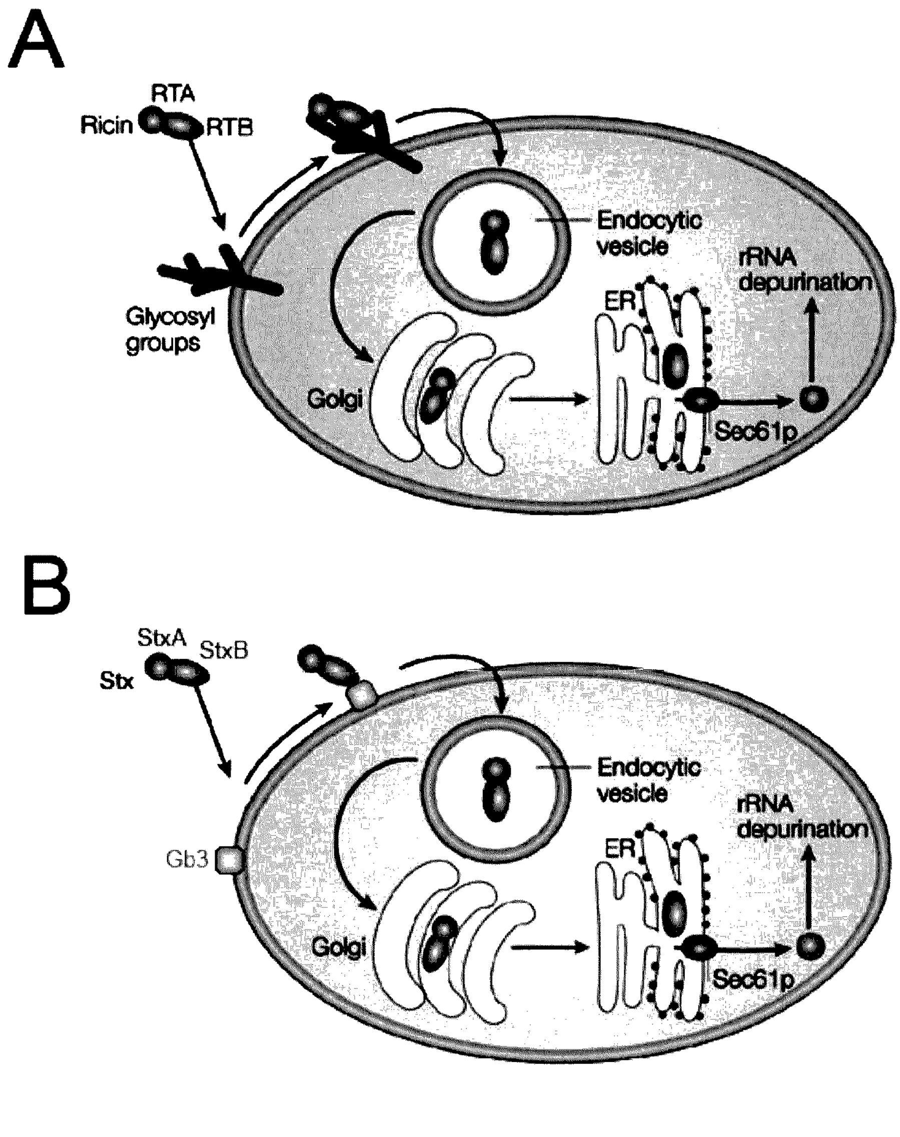


**Figure 2**

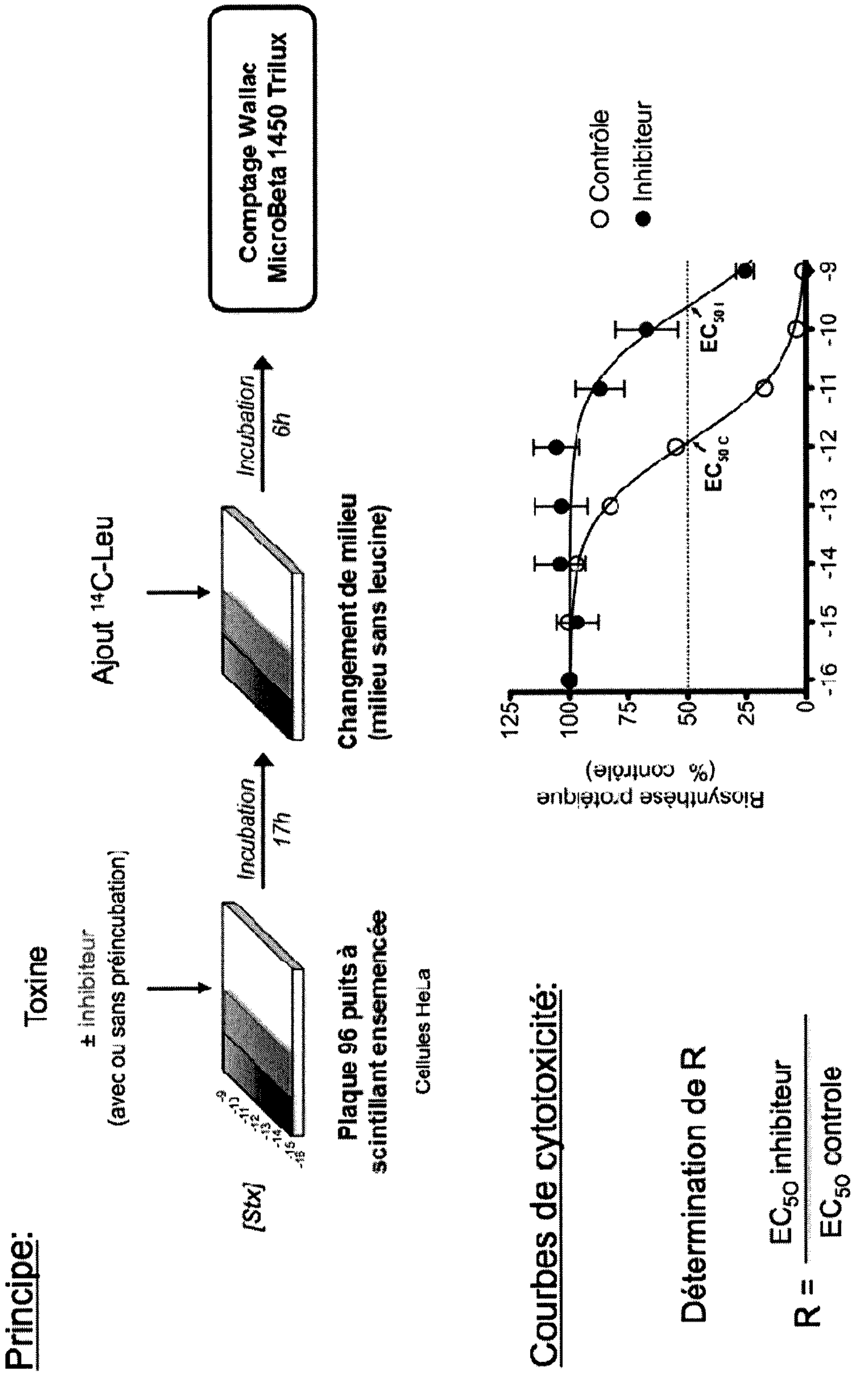
Principe:
[Stx]
-9
-10
-11
-12
-13
-14
-15
-16
Toxine
± inhibiteur
(avec ou sans préincubation)
Plaque 96 puits à scintillant ensemencée
Cellules HeLa
Incubation
17h
Ajout 14C-Leu
Changement de milieu
(milieu sans leucine)
Incubation
6h
Comptage Wallac
MicroBeta 1450 Trilux
Courbes de cytotoxicité:
Biosynthèse protéique
(% contrôle)
125
100
75
50
25
0
-16
-15
-14
-13
-12
-11
-10
-9
log Stx-1 (M)
EC50 C
EC50 I
O Contrôle
● Inhibiteur
Détermination de R
R = EC50 inhibiteur / EC50 controle

**Figure 3**

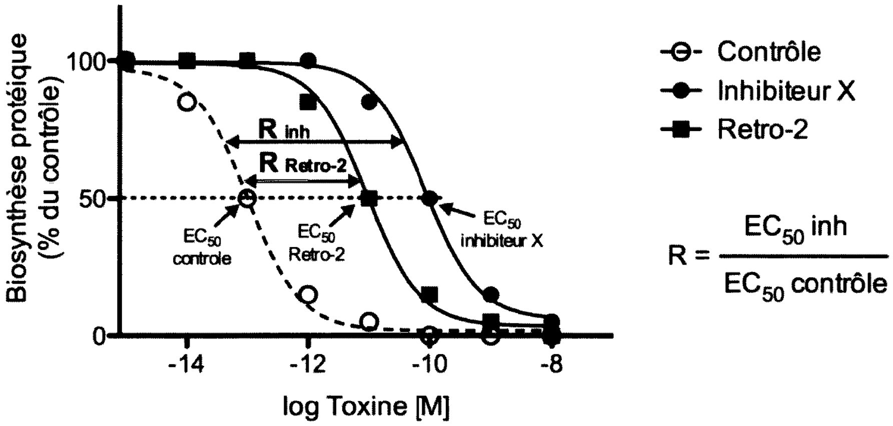


**Figure 4**

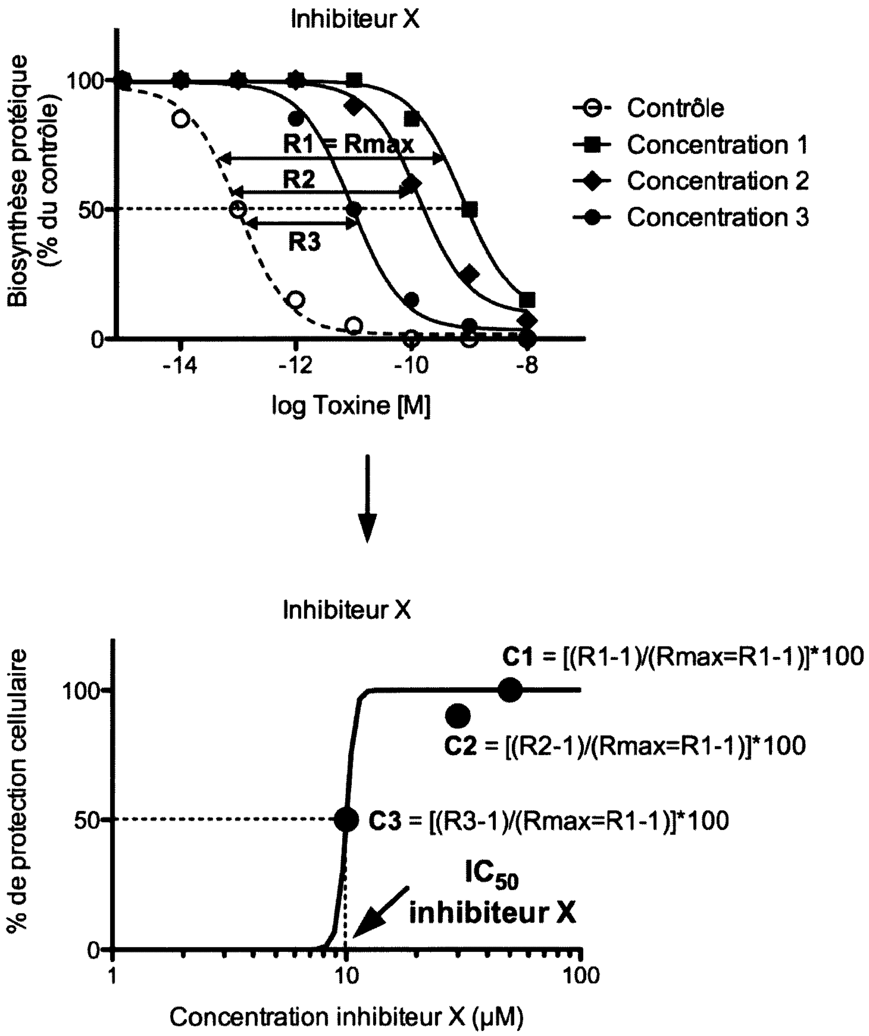


**Figure 5**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 2145873 A
- WO 2009153457 A
- WO 2009153665 A


### Littérature non-brevet citée dans la description


- **LORD, J.M. et al.** *Biochemical Society Transactions,* 2003, vol. 31, 1260
- **MILLER, D. et al.** *J. Med. Chem,* 2002, vol. 45, 90
- **YAN, X. et al.** *J. Mol. Biol.,* 1997, vol. 266, 1043
- **BRIGOTTI M. et al.** *Nucleic Acids Res.,* 2000, vol. 28 (12), 2383
- **SCHRAMM et al.** *Biochemistry,* 2001, vol. 40 (23), 6845
- **RODAY, S. et al.** *Biochemistry,* 2004, vol. 43, 4923
- **HESSELBERTH, J. R. et al.** *J. Biol. Chem.,* 2000, vol. 275, 4937
- **FAN, S. et al.** *World J. Gastroenterol.,* 2008, vol. 14, 6360
- **LEMLEY, P.V. et al.** *Hybridoma,* 1994, vol. 13, 417
- **GUO, J.W. et al.** *Hybridoma,* 2005, vol. 24, 263
- **PELAT, T. et al.** *BMC Biotechnol,* 2009, vol. 9, 60
- **SHEORAN A.S. et al.** *Infect Immun.,* 2005, vol. 73, 4607-13
- **TZIPORI S. et al.** *Clin. Microbiol. Rev.,* 2004, vol. 17, 926-41
- **PERERA L.P. et al.** *J Clin Microbiol.,* 1988, vol. 26, 2127-31
- **SMITH M.J. et al.** *Infect Immun.,* 2006, vol. 74, 6992-8
- **ARMSTRONG G.D. et al.** *J. Infect. Dis.,* 1995, vol. 171, 1042-5
- **TRACHTMAN H. et al.** *JAMA,* 2003, vol. 290, 1337-44
- **KITOV P.I. et al.** *Nature,* 2000, vol. 403, 669-72
- **NISHIKAWA K. et al.** *PNAS,* 2002, vol. 99, 7669-74
- **MULVEY G.L. et al.** *J. Infect. Dis.,* 2003, vol. 187, 640-9
- **WATANABE et al.** *J. Infect. Dis.,* 2004, vol. 189, 360-8
- **DONTA S.T. et al.** *J. Infect. Dis.,* 1995, vol. 171, 721-4
- **SPOONER R.A. et al.** *Biochem J.,* 2008, vol. 414, 471-84
- **YARROW J.C. et al.** *C.C.H.T.S.,* 2003, vol. 6, 279-86
- **SAENZ J.B. et al.** *Nat. Chem. Biol.,* 2009, vol. 5, 157-65
- **SAENZ J.B. et al.** *Infect. Imm.,* 2007, vol. 75, 4552-61
- **STECHMANN B. et al.** *Cell,* 2010, vol. 141, 231-42
- **WAHOME P.G. et al.** *Toxicon,* 2010, vol. 56, 313-23
- **PARK et al.** Chemical Structure of Retro-2, a Compound That Protects Cells against Ribosome-Inactivating Proteins. *Nature Scientific Reports,* 2012, vol. 2, 631
- **AMESSOU M. et al.** Curr. Protoc. Cell. Biol. 2006
- **STECHMANN B. et al.** *Cell,* 2010, vol. 41, 231-24